# FASCICULE DE BREVET EUROPEEN

(11) **EP 0 531 218 B1**
(45) Date de publication et mention de la délivrance du brevet: **18.07.2001**
(21) Numéro de dépôt: 92402414.4
(22) Date de dépôt: 04.09.1992
(51) Int. Cl.: C12N 15/56, C12N 9/24, A01H 5/10, C12N 1/15, C12N 1/19, C12N 5/10, A01H 5/00, C12N 1/21, C12N 9/42

(54) **ADN recombinant codant pour une protéine à activité endochitinase**
Rekombinant DNS kodierend für eine Endochitinase
Recombinant DNA coding for an endochitinase

(30) Priorité: 06.09.1991 FR 9111072
(43) Date de publication de la demande: 10.03.1993
(73) Titulaire: Biogemma, 75001 Paris (FR)
(72) Inventeur: Blaiseau, Pierre-Louis, F-94800 Villejuif (FR); Legoux, Richard, F-31460 Le Faget (FR); Leguay, Jean-Jacques, F-75012 Paris (FR); Schneider, Michel, F-31000 Toulouse (FR)
(74) Mandataire: Gillard, Marie-Louise

(56) Documents cités:
- EP-A- 0 290 123
- EP-A- 0 437 320
- WO-A-88/00976
- WO-A-90/07001
- EXPERIENTIA. vol. 41, 1985, BASEL CH pages 1612 - 1613 A. SRIVASTAVA ET AL 'Secretion of chitinase by Aphanocladium album, a hyperparasite of wheat rust'
- PROCEEDINGS OF THE NATIONAL ACADEMY OF SCIENCES OF USA. vol. 83, 1986, WASHINGTON US pages 6820 - 6824 K. BROGLIE ET AL 'Ethylene-regulated gene expression: Molecular cloning of the genes encoding an endochitinase from Phaseolus vulgaris'
- CHEMICAL ABSTRACTS, vol. 116, no. 15, 13 Avril 1992, Columbus, Ohio, US; abstract no. 145051, page 210 & CURR. GENET. vol. 21, no. 1, 1992, pages 61-66 P. BLAISEAU ET AL 'Cloning and expression of a chitinase gene from the hyperparasitic fungus Aphanocladium album'
- CHEMICAL ABSTRACTS, vol. 117, no. 2, 3 Août 1992, Columbus, Ohio, US; abstract no. 43227, C. KUNZ ET AL 'Purification and characterization of a chitinase from the hyperparasitic fungus Aphanocladium album' page 390 & PHYSIOL. MOL. PLANT PATHOL. vol. 40, no. 2, 1992, pages 117-131
- KUNZ, C.: "Purification and Characterization of a Chitinase from the hyperparasitic", PHYSIOLOGICAL AND MOLECULAR PLANT PATHOLOGY, , 1992, Vol. 40, no. , pages 117 à 131

## Description

L'invention concerne un nouvel ADN recombinant codant pour une nouvelle protéine à activité endochitinase ou pour un précurseur de cette dernière, une bactérie, une levure ou un champignon contenant cet ADN recombinant, une cellule végétale, une plante, ou une partie de plante, en particulier une semence végétale, qui contiennent cet ADN recombinant, un procédé pour rendre les plantes résistantes aux agents pathogènes tels que des champignons, des bactéries, ainsi que des arthropodes, notamment des insectes, et des nématodes, qui comprend une étape de transformation par ce gène, ainsi que cette nouvelle protéine et un procédé pour la préparer.

Les plantes cultivées sont soumises à des attaques par des agents pathogènes, tels que des champignons et des bactéries, ainsi que des arthropodes, notamment des insectes, et des nématodes, lesquelles sont responsables de pertes importantes de récoltes. Le principal moyen actuel de lutte contre ces agents réside dans l'utilisation de substances chimiques à activité fongicide ou bactéricide. On sait aujourd'hui que les plantes réagissent naturellement à ces attaques par divers mécanismes de défense, malheureusement en général à déclenchement trop tardif et d'intensité trop faible pour être suffisamment efficaces. L'un de ces mécanismes comprend l'induction d'une enzyme appelée chitinase EC 3.2.1.14. (A. Toppan et al., 1982, Agronomie, 2, 829-834). Cette induction peut être artificiellement stimulée par des substances, telles que l'éthylène, avec comme conséquence l'augmentation de la résistance aux agents pathogènes de la plante traitée (Boller T., 1988, Oxford Surveys of Plant Molecular and Cell Biology, 5, 145-174).

La chitine est un polymère linéaire polysaccharidique, constitué d'unités N-acétylglucosamine associées par des liaisons β-1,4. Celui-ci est un composé structural présent dans la paroi de la plupart des champignons pathogènes, dans l'exosquelette des arthropodes, en particulier des insectes, et dans l'enveloppe externe des oeufs et des cystes de nématodes. Les enzymes appelées chitinases sont capables de dégrader la chitine. On distingue parmi celles-ci deux groupes différents définis selon leur mode d'attaque de la chitine : les exochitinases capables de libérer l'unité N-acétylglucosamine située aux extrémités non réductrices des chaînes et les endochitinases capables de fragmenter les chaînes, qui sont les seules chitinases capables d'inhiber in vitro la croissance des hyphes mycéliennes (Roberts W.K. et al., 1988, Gen. Microbiol, 134, 169-176). Les chitinases végétales connues sont en grande majorité de type endo, contrairement aux chitinases bactériennes connues qui sont de type exo (Roberts W.K. et al., 1988, Gen. Microbiol, 134, 169-176).

Des séquences d'ADN codant pour des exochitinases bactériennes ont déjà été isolées et clonées (Jones J.D.G. et al., 1986 EMBO J., 5, 467-473 et Sundheim L. et al., 1988, Physiol. Molec. Plant Pathol., 33, 483-491). Le brevet US-A-4 751 081 décrit l'isolement et le clonage du gène complet codant pour la chitinase de Serratia marcescens ainsi que la transformation par ce gène des bactéries Pseudomonas fluorescens NZ130 et Pseudomonas putida MK280. Ces bactéries transformées sont capables de dégrader légèrement une chitine colloïdale dispersée dans le milieu de culture bactérien. Les travaux de Harpster M.H. et al., 1989, Nucl. Ac. Res., 17, 5395 ont montré que ce gène code pour une exochitinase, ce qui explique la faible efficacité de dégradation observée (cf. tableau 2, col. 13 et 14 de ce document). La publication de Jones J.D.G. et al. (1988), Mol. Gen. Genet., 212, 536-542, mentionne la transformation de plantes de tabac par Agrobacterium tumefaciens contenant un gène chimérique comprenant la partie codante de l'exochitinase de Serratia marcescens, sous contrôle de différents promoteurs végétaux. Ce document ne donne aucune indication sur l'éventuelle augmentation de la résistance aux pathogènes conférée par l'expression de cette exochitinase.

Des séquences d'ADN génomique et/ou d'ADN complémentaire codant pour certaines endochitinases végétales ont d'autre part été isolées et clonées (Broglie K.E., 1986, Proc. Ntl. Acad. Sci. USA, 83, 6820-6824 et Hedrick S.A., 1988, Plant Physiol., 86, 182-186).

La demande de brevet WO 90/07001 décrit la construction d'un plasmide portant un ADN complémentaire de l'endochitinase de haricot Phaseolus vulgaris sous le contrôle d'un promoteur fort, la transformation à l'aide d'Agrobacterium tumefaciens, la régénération du tabac transformé, des essais montrant la résistance accrue aux champignons, Rhizoctonia solani et Botrytis cinerea des plantes régénérées, l'obtention de plantes de tomates transgéniques exprimant la chitinase de haricot ainsi que l'obtention à l'aide de ce gène de plantes de colza transgéniques ayant un niveau d'activité chitinase et une résistance à Rhizoctonia solani accrus par rapport aux plantes de colza non transformées.

On n'a jamais isolé jusqu'à présent une séquence d'ADN génomique et/ou d'ADN complémentaire codant pour une chitinase de champignon filamenteux. La seule chitinase de champignon filamenteux partiellement isolée et caractérisée est l'endochitinase d'Aphanocladium album (Kuntz, 1991-Thèse de Doctorat de l'Université de P. et M. Curie-Paris).

Par ailleurs, la publication de Srivastava et al. (1985), Experentia, 41, 1612-1613, rapporte qu'Aphanocladium album sécrète de grandes quantités de chitinase sur un milieu synthétique contenant de la chitine, sans plus d'indications sur la nature de la chitinase purifiée.

La présente invention concerne un nouvel ADN recombinant caractérisé en ce qu'il code pour une protéine à activité endochitinase ou pour un précurseur de cette dernière, cette protéine à activité endochitinase comprenant la séquence d'acides aminés (a1) suivante (SEQ ID n° : 1) : ou une séquence présentant un degré d'homologie élevé avec la séquence (a1).

Un degré d'homologie élevé signifie ici une homologie (rapport entre les acides aminés identiques et le nombre total d'acides aminés) d'au moins 60 %, et de préférence d'au moins 80 %, des séquences d'acides aminés, lorsqu'elles sont alignées d'après l'homologie maximale, selon la méthode d'alignement optimal des séquences de Needleman et Wunsch, 1970, J. Mol. Biol, 48, 443-453. Cette méthode est notamment utilisée dans le logiciel UWGCG de l'Université du Wisconsin : Devereux et al., 1984, Nucl. Ac. Res., 12, 8711-8721 - option GAP.

La séquence peptidique de chitinase déjà connue la plus proche de la séquence (a1) est celle de la chitinase de Serratia marcescens (Jones J.D.G et al., 1986, EMBO J., 5, 467-473) avec une homologie d'environ 33 % (Kuntz, 1991, Thèse de Doctorat - Université P. et M. Curie, Paris) ; cette dernière chitinase est une exochitinase.

Cet ADN recombinant peut servir à l'expression de cette protéine à activité endochitinase, soit dans le but de conférer à une plante ou une partie de plante qui exprime celle-là, une résistance accrue aux agents pathogènes, soit dans le but de produire cette protéine à l'aide de cellules eucaryotes, notamment des ascomycètes tels que la levure par exemple Saccharomyces cerevisiae, des champignons filamenteux, par exemple, Cryphonectria parasitica, ou des cellules végétales, ou des micro-organismes procaryotes, tels que par exemple Escherichia coli.

Cet ADN recombinant comporte de préférence, en amont de la séquence codant pour la séquence (a1) ou de la séquence présentant un degré d'homologie élevé avec la séquence (a1), une séquence signal, choisie en fonction de la cellule hôte, qui a pour fonction de permettre l'exportation de la protéine hors du cytoplasme.

Pour une expression dans les micro-organismes procaryotes, tels que par exemple Escherichia coli, cette séquence signal peut être, soit une séquence dérivée d'un précurseur naturel d'une protéine exportée par un micro-organisme procaryote (par exemple le peptide signal OmpA (Ghrayeb et al., 1984, EMBO Journal, 3, 2437-2442) ou celui de la phosphatase alcaline (J. Bact. 1983, 154, 366-374)), soit une séquence non endogène provenant d'un précurseur eucaryote (par exemple le peptide signal de l'un des précurseurs naturels de l'hormone de croissance humaine), soit un peptide signal synthétique (par exemple celui décrit dans la demande de brevet FR n° 2 636 643).

Pour une expression dans les cellules eucaryotes telles que les ascomycètes, par exemple la levure Saccharomyces cerevisiae ou le champignon filamenteux Cryphonectria parasitica, cette séquence signal est de préférence une séquence dérivée d'un précurseur naturel d'une protéine sécrétée par ces cellules, par exemple pour la levure celui de l'invertase (demande de brevet EP 0 123 289) ou celui de la séquence prépro de la phéromone alpha (demande de brevet DK 2484/84), ou pour Cryphonectria parasitica, celui de la séquence prépro de l'endothiapepsine, décrit dans la demande de brevet EP 475 842, de séquence (SEQ ID n° : 2) :

Pour une expression dans les cellules végétales, on utilise comme séquence signal, soit une séquence codant pour le peptide signal d'une protéine de cellule végétale connue pour être exportée, par exemple, celle de l'endochitinase de tomate (Thèse de Doctorat es Sciences-spécialité : biologie moléculaire végétale, 1986, de M. Durant-Université de Paris Sud) de séquence (SEQ ID n° : 3) : ou celle de la chitinase de haricot (Broglie KE et al, 1986, Proc. Natl. Sci. USA, 83, 6820-6824) de séquence (a5) suivante (SEQ ID n° : 28) : soit une séquence signal codant pour le peptide signal de séquence (a2) suivante (SEQ ID n° : 4) : le peptide signal codé par la séquence signal pouvant éventuellement être séparé de la séquence (a1) dans la protéine codée par un ou plusieurs acides aminés, en particulier par le peptide de séquence (a3) suivante (SEQ ID n° : 5 ) :

Les séquences d'acides aminés (a1), (a2) et (a3) peuvent par exemple être codées par les séquences nucléotidiques (Na1), (Na2) et (Na3) ci-après.

La séquence d'acides aminés (a5) peut par exemple être codée par la séquence nucléotidique (Na5) suivante (SEQ ID n° : 29) :

L'invention concerne aussi une unité d'expression de l'ADN recombinant défini précédemment, portée avantageusement par un vecteur, dit vecteur d'expression.

Pour une expression dans les micro-organismes procaryotes, en particulier dans Escherichia coli, l'ADN recombinant doit être inséré dans une unité d'expression comportant notamment un promoteur efficace, suivi d'un site de fixation des ribosomes en amont du gène à exprimer, ainsi qu'une séquence d'arrêt de transcription efficace en aval du gène à exprimer. Cette unité doit également comporter un marqueur de sélection ou être introduite dans la cellule hôte en même temps qu'une unité d'expression d'un marqueur de sélection (par exemple à l'aide d'un vecteur d'expression qui porte ces deux unités). Toutes ces séquences doivent être choisies en fonction de la cellule hôte.

Pour une expression dans les cellules eucaryotes telles que les ascomycètes, l'unité d'expression selon l'invention comprend l'ADN recombinant défini précédemment avec les moyens nécessaires à son expression.

Pour une expression dans les cellules ascomycètes telles que la levure, par exemple Saccharomyces cerevisiae, il convient d'insérer l'ADN recombinant entre, d'une part, des séquences reconnues comme promoteur efficace, d'autre part, un terminateur de transcription. L'unité d'expression porte un marqueur de sélection ou est introduite dans la cellule hôte en même temps qu'un marqueur de sélection. De préférence, ce marqueur de sélection est un marqueur auxotrophe (qui complémente une mutation des cellules réceptrices) qui permet la sélection des cellules qui ont intégré l'ADN recombinant en un nombre de copies élevé, soit dans leur génome, soit dans un vecteur multicopie.

Pour une expression dans les cellules ascomycètes telles que celles des champignons filamenteux, par exemple ceux des genres Aspergillus, Neurospora, Podospora, Trichoderma ou Cryphonectria, l'unité d'expression selon l'invention porte l'ADN recombinant défini précédemment avec les moyens nécessaires à son expression, et éventuellement un marqueur de sélection et/ou des séquences télomériques. Il est en effet possible de sélectionner les transformants ayant intégré un ADN d'intérêt à l'aide d'un marqueur de sélection situé soit sur la même unité que l'ADN d'intérêt, soit sur une autre unité, ces deux unités étant alors introduites par cotransformation. L'ADN recombinant de l'invention peut être, soit intégré au génome des champignons filamenteux, soit conservé sous forme extrachromosomique grâce à des séquences permettant la réplication et la partition de cet ADN.

Pour une expression dans les cellules végétales, il convient d'insérer l'ADN recombinant défini précédemment entre un promoteur et un terminateur efficaces dans les végétaux.

Le promoteur est de préférence un promoteur constitutif fort, par exemple le promoteur 35S du virus de la mosaïque du chou-fleur, ou un promoteur commandant une expression tissu ou organe spécifique comme le promoteur de la petite sous-unité de la ribulose 1,5-bisphosphate carboxylase-oxygénase qui s'exprime préférentiellement dans les feuilles et tout particulièrement les tissus du mésophylle (Kuhlemeier et al., 1987, Ann Rev Plant Physiol 38 : 221-257). On peut également utiliser un promoteur spécifique commandant par exemple une expression dans les graines ou au cours d'un stade précis du développement de la plante, ou un promoteur inductible à la suite d'un choc thermique, d'une blessure ou de l'interaction entre la plante et des parasites (Kuhlemeier et al., 1987 référence citée ci-dessus) si une expression de l'ADN recombinant est recherchée dans ces situations.

On utilise la séquence terminatrice, comportant des sites de polyadénylation, pouvant être isolée de gènes végétaux ou de gènes s'exprimant dans les végétaux, comme par exemple le terminateur de la nopaline synthase d'Agrobacterium tumefaciens.

L'invention concerne également une bactérie, par exemple de l'espèce Escherichia coli, une levure, par exemple Saccharomyces cerevisiae ou un champignon filamenteux, par exemple Cryphonectria parasitica ou Fusarium oxysporum, qui contient l'ADN recombinant défini précédemment, avec les moyens nécessaires à sa réplication et son expression. Cette bactérie, cette levure ou ce champignon filamenteux peut être utilisé dans la préparation d'une protéine à activité endochitinase.

L'invention a également trait à une bactérie, par exemple de l'espèce Escherichia coli, qui contient l'ADN recombinant défini ci-dessus avec les moyens permettant sa réplication, laquelle donc, peut servir au clonage de cet ADN recombinant, ainsi qu'à une bactérie susceptible d'infecter une plante avec transfert de matériel génétique, par exemple de l'une des espèces Agrobacterium rhizogenes et Agrobacterium tumefaciens, qui contient cet ADN dans un contexte permettant sa réplication et donc peut servir à transformer des cellules végétales. La transformation des cellules végétales par l'ADN recombinant ci-dessus peut également être effectuée par une autre méthode biologique telle que la voie du tube pollinique (Zhong-xun Luo et al., Plant Molec. Biol. Rep., 1988, 6, 165-176) et la transformation directe de graines en germination (Toepfer R. et al., 1989, The Plant Cell., 1, 133-139), ou par une méthode physique telle que l'utilisation de polyéthylèneglycol, l'électroporation (Chistou P. et al., 1987, Proc. Ntl. Acad. Sci. USA, 84, 3662-3699) et le bombardement à l'aide de microprojectiles (Klein T.M. et al., 1988, Proc. Ntl. Acad. Sci. USA, 85, 8502-8505).

L'invention concerne également une cellule végétale, caractérisée en ce qu'elle est transformée par l'ADN recombinant défini précédemment, avec les moyens nécessaires à son expression. Cette cellule végétale peut provenir d'une espèce de grande culture, telle que par exemple le maïs, le soja, la betterave, le blé, l'orge, le pavot, le colza, le tournesol, la luzerne et le sorgho, d'une espèce florale telle que le rosier, l'oeillet, le gerbera ou d'une espèce potagère telle que la carotte, la tomate, la salade, la chicorée, le poivron, le melon et le chou. Des espèces particulièrement appréciées sont le colza Brassica napus, le tournesol Helianthus annuus et le tabac Nicotiana tabacum.

L'étape de transformation qui concerne une ou quelques cellules est suivie d'une étape de multiplication de ces cellules transformées de façon à obtenir des cals, lesquels peuvent donner naissance à des plantes transformées, par des processus d'organogénèse ou d'embryogénèse.

L'invention concerne donc aussi une plante ou une partie de plante, caractérisée en ce qu'elle contient, avec les moyens nécessaires à son expression, l'ADN recombinant défini précédemment. Une partie de plante particulièrement appréciée est la partie apte à former une nouvelle plante complète, notamment après semis, enfouissement ou repiquage, ou à produire des semences. Une telle partie est par exemple un grain, une graine, une semence, une bouture, une marcotte, etc. Ces plantes peuvent être l'une quelconque des espèces ci-dessus et plus particulièrement des espèces Nicotiana tabacum, Helianthus annuus et Brassica napus.

L'invention a également trait à un procédé pour obtenir des plantes résistantes aux parasites, tels que les champignons phytopathogènes et les bactéries ainsi que les arthropodes, notamment les insectes, et les nématodes, qui comprend une étape de transformation de cellules végétales par cet ADN recombinant, suivie d'une étape de multiplication des cellules transformées et d'une étape de régénération des plantes.

De préférence, l'étape de transformation des cellules végétales est effectuée in vitro à l'aide d'une agrobactérie (c'est-à-dire d'une bactérie du genre Agrobacterium) ayant intégré l'ADN recombinant d'intérêt.

L'invention concerne aussi les plantes résistantes aux agents pathogènes susceptibles d'être obtenues à l'aide du procédé défini ci-dessus.

L'invention a également trait à l'utilisation d'une plante contenant, avec les moyens nécessaires à son expression, l'ADN recombinant défini précédemment, comme géniteur dans un programme de sélection pour créer de nouvelles variétés végétales.

L'invention concerne aussi une nouvelle protéine à activité endochitinase, susceptible d'être obtenue à l'aide de l'ADN recombinant défini précédemment. Cette protéine comprend de préférence la séquence (a1) ou une séquence présentant un degré d'homologie élevé avec la séquence (a1). Elle a avantageusement un poids moléculaire apparent de 39 + 3 ou 41 + 3 kDa. Elle peut être N-glycosylée si elle est exprimée dans une cellule qui permet la glycosylation.

Cette protéine présente un intérêt comme principe actif d'un nouveau médicament destiné à soigner des affections, telles que par exemple les mycoses.

L'invention a également trait à un procédé de préparation de cette protéine qui comprend la mise en culture de cellules végétales, de cals végétaux, de plantes ou de parties de plantes contenant l'ADN recombinant défini précédemment, la lyse de ceux-ci, l'isolement et la purification de cette protéine.

L'invention sera mieux comprise à l'aide de l'exposé ci-après, divisé en sections, qui comprend des résultats expérimentaux et une discussion de ceux-ci. Certaines de ces sections concernent des expériences effectuées dans le but de réaliser l'invention, d'autres des exemples de réalisation de l'invention, donnés bien sûr à titre purement illustratif.

Une grande partie de l'ensemble des techniques ci-après, bien connues de l'homme de l'art, est exposée en détail dans l'ouvrage de Sambrook et al.: "Molecular Cloning : a Laboratory manual" publié en 1989 par les éditions Cold Spring Harbor Press à New York (2^{e} édition).

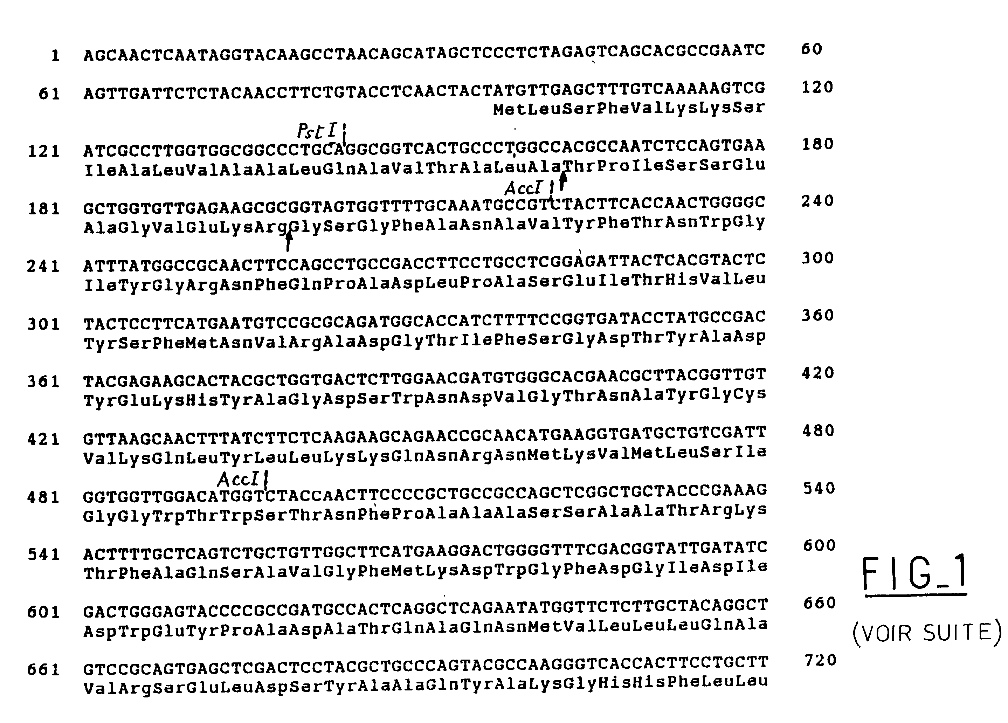

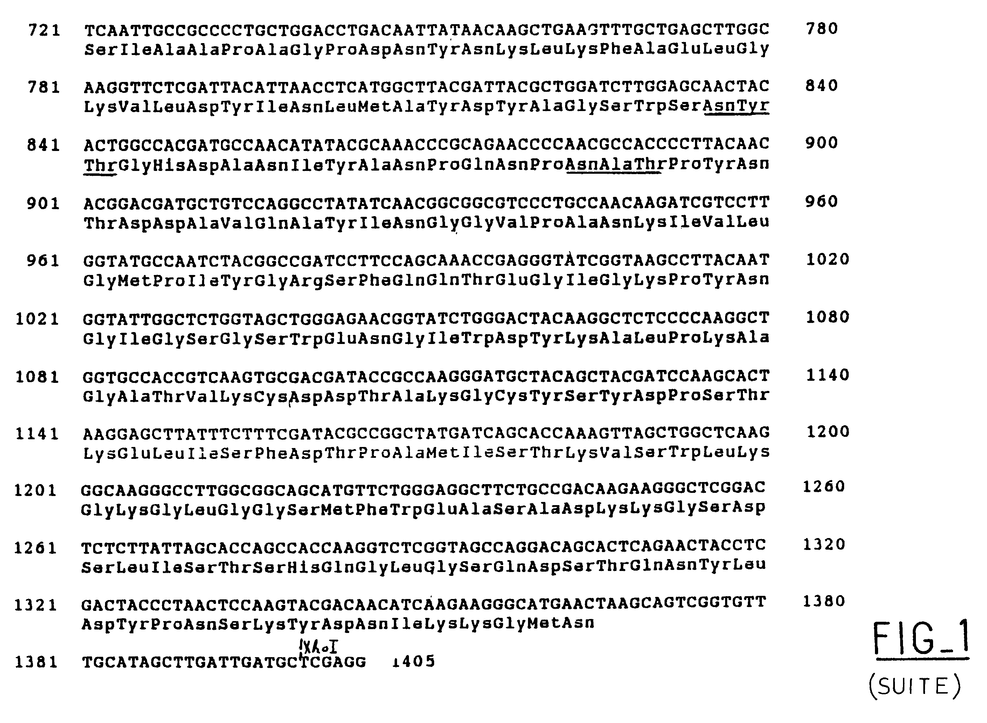

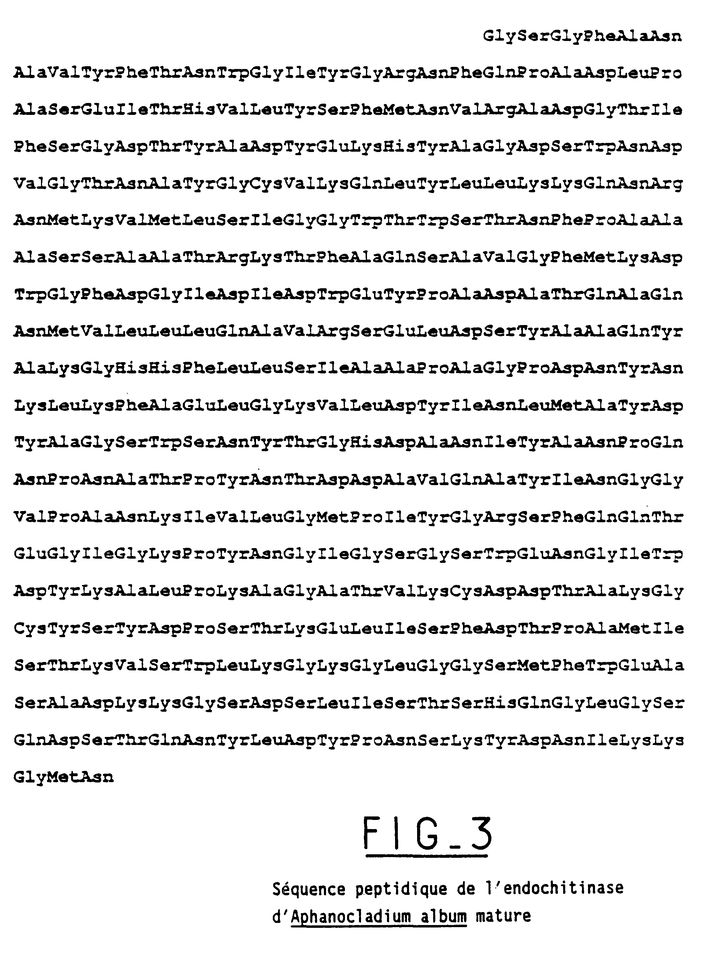

La description ci-après sera mieux comprise en se rapportant aux figures 1 à 5.

La figure 1 (SEQ ID n° : 9) représente la séquence nucléotidique de l'ADN complémentaire pleine longueur codant pour la chitinase d'Aphanocladium album et la séquence peptidique de la protéine traduite, le site de clivage entre la séquence peptidique pré et la séquence peptidique pro, ainsi que le site de clivage entre la séquence peptidique pro et la protéine mature étant représentés par des flèches en dessous de la séquence peptidique, les différents sites de restriction utilisés dans les constructions ultérieures étant indiqués par une barre verticale en pointillé au-dessus de la séquence nucléotidique, et les sites potentiels de N-glycosylation étant soulignés.

La figure 2 (SEQ ID n° : 11) représente la séquence nucléotidique de l'ADN génomique codant pour la chitinase d'Aphanocladium album, les nucléotides des introns étant représentés en minuscules, et la séquence peptidique de la protéine traduite.

La figure 3 (SEQ ID n° : 1) représente la séquence peptidique de la protéine mature.

Les figures 4 et 5 (SEQ ID n° 13-14) représentent les séquences codantes de la chitinase d'A. album dans respectivement les plasmides pBR61 et pBR62 (vecteurs d'expression dans les cellules végétales), encadrées par les sites de restrictions BamHI et SacI.

### Section 1 : Préparation des anticorps contre la chitinase d'Aphanocladium album

### a) Purification de la chitinase d'Aphanocladium album

Une chitinase du champignon filamenteux Aphanocladium album a été purifiée jusqu'à homogénéité à partir de milieu de culture d'A. album comme décrit ci-après :

La souche d'Aphanocladium album utilisée est le mutant surproducteur de chitinase E₃ obtenu par mutagénèse UV de la souche sauvage ETHM 483 selon le protocole décrit par Vasseur et al., 1990, J. Gen. Microbiol., 136, 12, 2561-2568. Cette souche a été cultivée sur un milieu de malt-agar dans les conditions décrites par Forrer H.R., Phytopath. Z. 88, (1977), 306. Des fragments prélevés de cette culture servent à ensemencer un milieu de culture liquide contenant 1 % de chitine (Srivastava A.K. et al., Experientia 41, (1985), 1612-1613). La protéine est purifiée à partir du milieu de culture par chromatographie en phase liquide selon la techyique FPLC de Pharmacia sur la colonne échangeuse de cations à base de polymère synthétique (Mono S de Pharmacia), et chromatographie d'exclusion (de tamisage moléculaire) sur un agarose réticulé selon le protocole décrit ci-après :

### Etape 1 :

Le milieu de culture est concentré 40 fois contre du polyéthylèneglycol (Carbowax 20 M-Touzart et Matignon), puis dialysé pendant une nuit à 4°C contre une solution tampon d'acétate de sodium 100 mM de pH 5,0. La quantité totale de protéines est déterminée par la méthode de Bradford (Bradford M.M, 1976, Anal. Biochem., 72, 248-254).

### Etape 2 :

Le milieu de culture concentré est ensuite fractionné par chromatographie sur une colonne d'échange d'ions à base de polymère synthétique (colonne Mono S de Pharmacia) selon la technique FPLC de Pharmacia. L'extrait, préalablement dilué dans une solution tampon d'acétate de sodium 10 mM de pH 5,2, est déposé sur la colonne MONO S (HR 5/5), équilibrée avec un tampon acétate de sodium 10 mM de pH 5,2. Les protéines retenues sur la colonne sont éluées par un gradient linéaire de 10 à 500 mM d'acétate de sodium pH 5,2.

### Etape 3 :

Les fractions contenant la chitinase d'Aphanocladium album sont concentrées par ultrafiltration sur membrane Centricon 10 (Amicon). La purification de la protéine est poursuivie par chromatographie d'exclusion sur un agarose réticulé (colonne Superose 12 Pharmacia), l'élution étant réalisée par une solution tampon d'acétate de sodium 500 mM de pH 5,2.

A chaque étape, la chitinase est identifiée par son poids moléculaire (électrophorèse sur gel de polyacrylamide à 12,5 % en présence de SDS - révélation à l'argent) et son activité enzymatique, mesurée par la méthode radiochimique décrite ci-après utilisant la chitine marquée au tritium comme substrat (Molano et al. (1977) Anal. Biochem 83, 648-656).

A l'issue de la purification, on isole une protéine de poids moléculaire apparent de 41 + kDa qui présente une activité endochitinase (thèse de C. Kuntz, Université de Pierre et Marie Curie, 1991). Cette protéine possède une activité chitinolytique mesurée par la méthode radiochimique au tritium décrite ci-après.

### b) Caractérisation de la chitinase d'Aphanocladium album

### b1. Mesure de l'activité enzymatique de la chitinase d'Aphanocladium album

L'activité endochitinase de la chitinase est mesurée par une méthode radiochimique permettant d'estimer la quantité de monomères ou d'oligomères libérés par l'enzyme à partir d'un substrat (la chitine tritiée). Cette méthode, décrite par Molano et al. (1977, Anal. Biochem., 83, 648-656), est résumée ci-après.

A un volume d'extrait protéique de 10 µl sont ajoutés 50 µl de chitine tritiée d'activité spécifique 0,58 MBq/ml. Le volume final est ajusté à 300 µl avec du tampon d'acétate de sodium 0,2 M de pH 5,0. Après 90 min d'incubation à 30°C, la réaction d'hydrolyse de la chitine est arrêtée par 100 µl d'acide trichloracétique à 20 %. Les tubes réactionnels sont ensuite centrifugés pendant 10 min à 12 000 g. Une partie aliquote de 100 µl du surnageant renfermant les oligomères solubles de chitine est prélevée et la radioactivité correspondante est mesurée par scintillation liquide en présence de 5 ml de mélange scintillant. On exprime l'activité chitinolytique spécifique en dpm/µg de protéine.

### b2. Détermination de la séquence aminoterminale de la chitinase d'Aphanocladium album

Le séquençage de l'extrémité aminoterminale de la protéine isolée a été réalisé comme décrit ci-après. Les échantillons à traiter sont portés à la surface d'un filtre PVDF ("polyvinylidènedifluoride" : difluorure de polyvinylidène) qui est introduit dans un séquenceur de protéines (Modèle 470 A, commercialisé par la Société Applied Biosystems E.U.A.) équipé d'un chromatographe (Modèle 430 d'Applied Biosystems) qui analyse en continu les dérivés phénylthiohydantoïques formés après chaque cycle de dégradation.

La séquence aminoterminale déterminée est la séquence suivante (acides aminés 1-23 de SEQ ID n° : 1) :

### c) Préparation d'anticorps polyclonaux

Pour préparer un immunsérum, on a injecté à des lapins 25 µg de chitinase purifiée dans 500 µl d'adjuvant complet de Freund. Trois injections de rappel de 25 µg dans l'adjuvant incomplet de Freund (500 µl) ont été réalisées à 3 semaines d'intervalle. L'immunsérum a été prélevé 3 semaines après la dernière injection.

Cet immunsérum reconnaît spécifiquement la chitinase d'Aphanocladium album. Il permet de révéler cette dernière protéine notamment par la technique de Western Blot (décrite dans la section 8) à partir d'un extrait de protéines totales d'une souche d'Aphanocladium album cultivée dans les conditions décrites ci-dessus.

### Section 2 : Construction de la banque d'ADN complémentaire d'Aphanocladium album

### a) Préparation d'ARN messagers extraits d'Aphanocladium album

Les ARN totaux de mycélium de la souche d'Aphanocladium album précédente cultivée pendant 2 jours sur un milieu en présence de 1 % de chitine, ont été extraits selon la méthode de Logeman et al., Analytical Biochemistry, 1987, 163, 16-20.

Le mycélium est séparé du milieu de culture par filtration, lavé par de l'eau stérile, puis broyé au mortier dans de l'azote Liquide ; les ARN totaux sont ensuite extraits par la méthode à l'hydrochlorure de guanidine en suivant les recommandations de Logeman et al. (réf. ci-dessus). Après une étape de précipitation éthanolique, les ARN totaux sont dissous dans une solution tamponnée.

Les ARN messagers poly (A)⁺ ont été isolés après 2 cycles de chromatographie sur une colonne d'oligo (dT) cellulose comme décrit dans Sambrook et al., (op cité). La quantification des ARN messagers (ARNm) est réalisée par spectrophotométrie à 260 nm.

### b) Synthèse des ADN complémentaires

Les ADN complémentaires ont été synthétisés à l'aide du kit "Riboclone cDNA Synthesis System" de Promega (réf : C2100) en suivant les recommandations du fournisseur. Ce kit utilise la méthode décrite par Okayama et al., 1982, Mol. and Cell. Biol. 2, 161-170 et modifiée par Gübler et Hoffman, 1983, Gene, 25, 263-269, qui favorise la synthèse et le clonage d'ADNc complets. Les ADN complémentaires ont été clonés au site EcoRI dans le vecteur λgt11 en suivant la procédure du système de clonage d'Amersham (cDNA cloning system kit, réf. RPN 1280). Le nombre de recombinants a ensuite été estimé par comptage des plages de lyse obtenues sur un tapis de bactéries de la souche E. coli Y 1090 (Sambrook et al., op. cité). Environ 10⁵ clones ont été obtenus, dont 80 % sont des clones recombinants.

### Section 3 : Criblage immunologique de la banque d'ADN complémentaires construite à partir des ARN messagers d'Aphanocladium album

La réalisation d'une banque dans le vecteur gt11 permet de réaliser l'expression des ADNc clonés sous forme de protéines codées par les ARNm qui ont servi à construire cette banque. Cette synthèse se fait après induction à l'isopropyl thio-β-D-galactoside (IPTG) ; les protéines synthétisées peuvent alors être reconnues par des anticorps préalablement obtenus contre la protéine recherchée (section 1). Les clones peuvent être repérés et isolés selon un protocole connu de l'homme de l'art et décrit par exemple par Sambrook et al. (op. cité).

La banque est amplifiée par infection de la souche E. coli Y 1090 à l'aide d'une suspension de phages contenant 10⁴ particules capables de former des plages de lyse. Cette étape est réalisée dans des boîtes de Pétri de 90 mm de diamètre. L'incubation est réalisée à 42°C pendant 16 h et permet une amplification de 10⁷ fois.

La banque amplifiée est ensuite étalée sur un tapis bactérien de la souche E. coli Y 1090 à une densité de 10⁵ particules capables de former des plages de lyse. Les bactéries sont étalées dans 5 boîtes de Pétri de 150 mm de diamètre et incubées à 42°C pendant 5 h. Un filtre de cellulose (Schleicher et Schuell, BA 85) imprégné d'IPTG (10 mM) est déposé sur la surface des boîtes et laissé en contact avec le milieu gélosé pendant 5 h à la température de 37°C, puis remplacé par un second filtre qui est laissé pendant 16 h sur le même milieu. Les filtres sont traités par un tampon dit de blocage composé de 10 % de lait en poudre (Regilait) dans du tampon TNT (10 mM Tris pH 8,0, 150 mM NaCl, 0,05 % Tween) pendant 30 min et incubés avec l'antisérum décrit précédemment, dilué au 1/100 dans le milieu TNT. Le protocole d'incubation et de révélation à la phosphatase alcaline est celui décrit par le "Protoblot immunoscreening system" de Promega réf. S3710. Les clones positifs apparaissent colorés en bleu-violet après révélation.

Les plages de lyse positives, c'est-à-dire correspondant à des clones qui synthétisent la chitinase, sont alors repérées sur la boîte de Pétri et les bactériophages sont prélevés pour être purifiés grâce à un criblage immunologique secondaire, conduit de façon strictement identique au criblage primaire qui vient d'être décrit. Dix-sept clones ont été obtenus correspondant à 7 groupes d'hybridation différents. Un groupe d'hybridation de 9 clones d'environ 230 pb fournit un signal particulièrement fort. L'un de ces clones, dénommé CH3C, a été retenu pour la suite de l'étude.

La séquence de l'ADN du clone CH3C a été déterminée selon la méthode des désoxyribonucléotides (Sanger et al., 1977, Proc. Ntl. Acad. Sci. USA, 14, 5463-5467). Ce clone ne renferme pas l'ADN complémentaire "pleine longueur", car sa taille est trop petite.

### Section 4 : Construction de la banque d'ADN génomique d'Aphanocladium album

### a) Préparation et encapsidation de l'ADN total d'Aphanocladium album

L'ADN total a été préparé par la méthode de Daboussi et al. Curr. Genet., (1989), 15, 453-456. L'ADN d'A. album a ensuite été partiellement digéré par l'enzyme de restriction Sau3AI et fractionné selon la taille sur un gradient de saccharose. Les fragments d'une taille comprise entre 12 et 20 kb ont été insérés dans le phage EMBL4 après coupure de ce dernier au site BamHI. L'encapsidation dans les particules phagiques est réalisé in vitro en utilisant le kit "cDNA cloning system" d'Amersham, réf : RPN.1280, et la transfection est réalisée sur un tapis de bactéries de la souche E. coli LE 292 (Sambrook et al., op cité). La taille de la banque est de 1,2 x 10⁶ plaques et possède 50 % de phages recombinants.

### b) Criblage de la banque génomique d'Aphanocladium album

### b1. Préparation des répliques sur filtre

Après amplification de la banque par infection de la souche E. coli NM 539 (Sambrook et al., op cité) selon les techniques connues de l'homme de l'art, on étale 10⁵ phages à une densité de 20 000 particules capables de former des plages de lyse par boîte sur un tapis de bactéries de la souche NM 539. L'incubation dure 16 h à 37°C. Les boîtes sont refroidies et 2 répliques sont effectuées en déposant successivement 2 filtres de Nylon (Hybond N, Amersham réf. RPN 203N). Le premier est laissé en contact avec les plages de lyse pendant 45 s, le second pendant 90 s.

Les répliques sur membrane sont déposées avec l'ADN vers le haut, sur une feuille de papier Whatman 3MM saturée avec une solution de dénaturation de composition : NaOH 0,5 M ; NaCl 1,5 M, pendant 7 min, ce qui permet de fixer l'ADN. Les répliques sur membrane sont ensuite posées sur une seconde feuille de Whatman 3MM saturée cette fois avec une solution neutralisante de composition : NaCl 1,5 M, Tris-HCl pH 7,4 0,5 M, pendant 3 min. Les répliques sur membranes sont ensuite plongées dans une solution de 2 x SSC (NaCl 0,30 M ; citrate de sodium 0,030 M), puis séchées à l'air libre, la face ayant fixé l'ADN étant dirigée vers le haut.

### b2. Préparation de la sonde radioactive utilisée pour repérer les clones positifs et hybridation des répliques

La sonde utilisée est l'ADN du clone CH3C obtenu précédemment (section 3), dont 100 ng d'ADN sont marqués à l'aide de dCTPα³²P (3 000 Ci/mmol Amersham) par marquage au hasard ("random priming") en utilisant le kit de marquage de Boehringer Mannheim GmbH (réf : 1004 760), utilisé selon les recommandations du fabricant. L'activité spécifique obtenue est de 1 x 10⁹ dpm/µg d'ADN.

Les répliques sur membrane sont préhybridées pendant 1 h à 65°C dans un tampon de composition : 6 x SSC ; 5 x solution de Denhardt ; 0,5 % SDS et 100 µg/ml d'ADN de sperme de saumon soniqué. Les répliques sur membrane sont hybridées avec la sonde préparée précédemment pendant 16 h dans le même tampon, puis sont lavées pendant 20 min à 20°C dans un tampon 2 x SSC ; 0,1 % SDS, puis pendant 40 min dans un tampon 2 x SSC ; 0,1 % SDS à 65°C, et enfin pendant 40 min dans un tampon 0,2 x SSC ; 0,1 % SDS à 65°C, puis séchées et autoradiographiées. En résumé, le tampon 20 x SSC contient 175,3 g/l de NaCl ; 88,2 g/l de citrate de sodium et est ajusté à pH 7 par quelques gouttes de NaOH 10N. La solution 10 x Denhardt contient 1 g de Ficoll 400, 1 g de polyvinylpyrrolidone, 1 g d'albumine sérique bovine pour 500 ml de volume final. Cinq phages ont été purifiés en trois étapes. Quatre d'entre eux présentent un profil de digestion identique pour les enzymes de restriction EcoRI, HindIII et BamHI.

### b3. Clonage et séquençage d'un fragment renfermant un gène d'Aphanocladium album codant pour une chitinase

L'ADN de l'un de ces 4 phages a été digéré par l'enzyme BamHI. Les fragments de restriction obtenus ont été séparés par électrophorèse sur gel d'agarose 1 %. L'ADN a été transféré selon la méthode de Southern blot (Southern, E.M. (1975), J. Mol. Biol., 98, 503-517) sur un filtre de Nylon (Hybond N⁺ Amersham) et hybridé avec l'ADN du clone CH3C marqué au dCTPα³²P (cf. section 4 b2). Les membranes sont ensuite lavées et révélées selon le protocole recommandé par Amersham et autoradiographiées avec un film XAR (Kodak).

Un signal d'hybridation très fort sur une bande unique est mis en évidence. La taille de ce fragment d'ADN est estimée à environ 7kb. Ce fragment, appelé fragment fBL1, est ensuite isolé par électroélution après électrophorèse selon la méthode décrite dans l'ouvrage "Plant Molecular Biology Manual", Gelvin et al., Kluwer Academic Press, 1988, puis ligué dans un vecteur pUC13 ouvert au site BamHI et déphosphorylé (Pharmacia réf. : 27-4969-01). Le plasmide obtenu est appelé plasmide pBL1. Ce vecteur est introduit dans E. coli (DH5 αF') selon le protocole décrit par Sambrook et al., op cité. Le clone obtenu est appelé BL1.

### Séquençage :

Après une préparation de l'ADN double brin, selon les techniques connues de l'homme de l'art, une partie de l'insert de 7 kb a été séquencée selon la méthode de didéoxyribonucléotides (Sanger et al., Proc. Ntl. Acad. Sci. USA, 14, 5463-5467, 1977) à l'aide du kit de Pharmacia "T⁷ sequencing kit", réf : 27-1 682-01.

On utilise comme amorce le mélange des oligonucléotides de formule ci-après, appelé mélange d'oligonucléotides N₁, correspondant aux séquences déduites de la séquence aminoterminale de la protéine purifiée (cf. section 1). Ce mélange a été obtenu par synthèse chimique à l'aide d'un appareil Biosearch 4600. (I représente l'inosine)

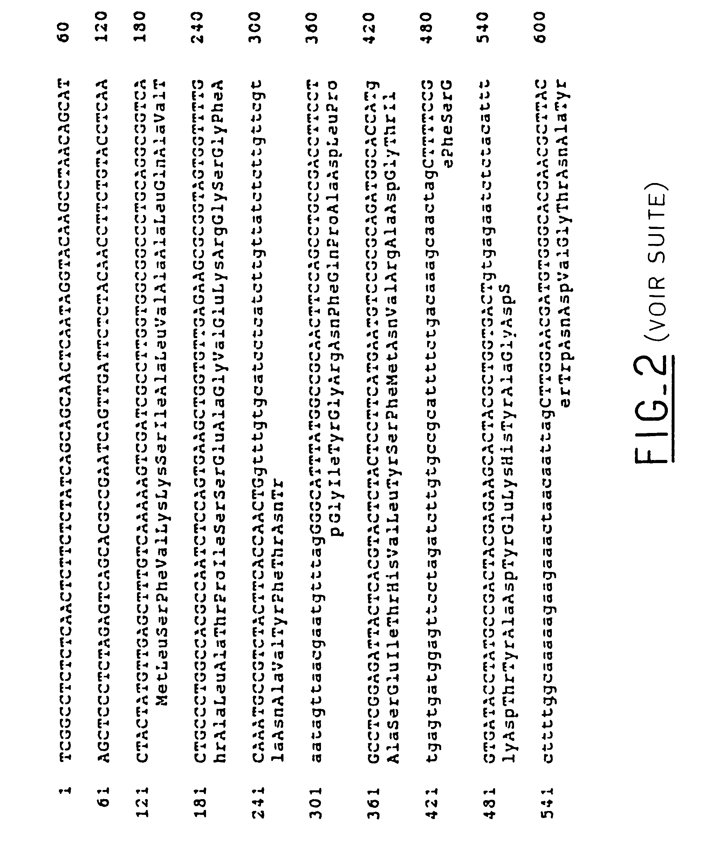

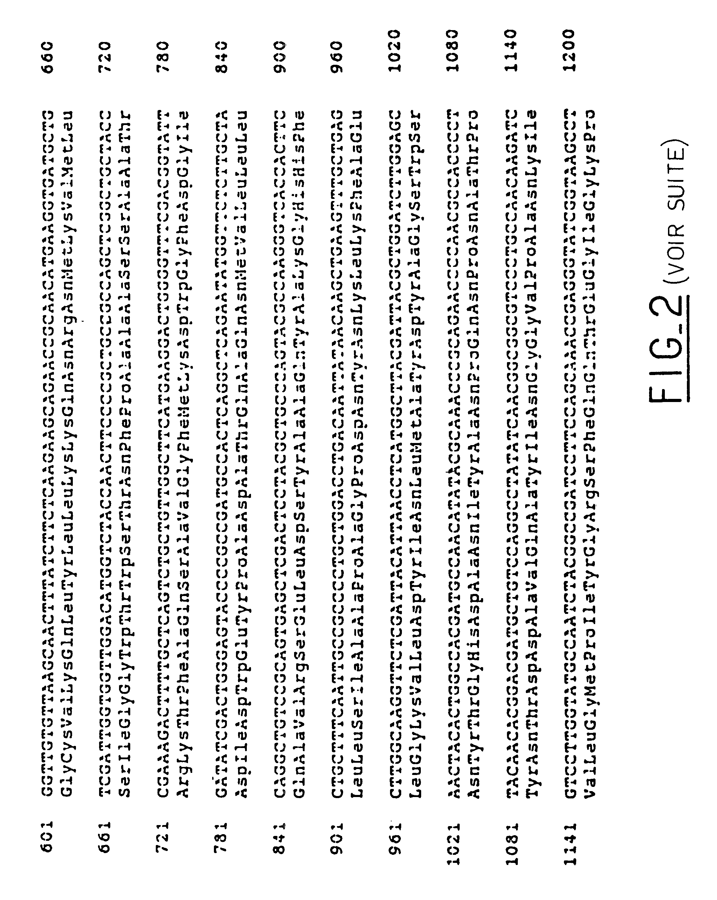

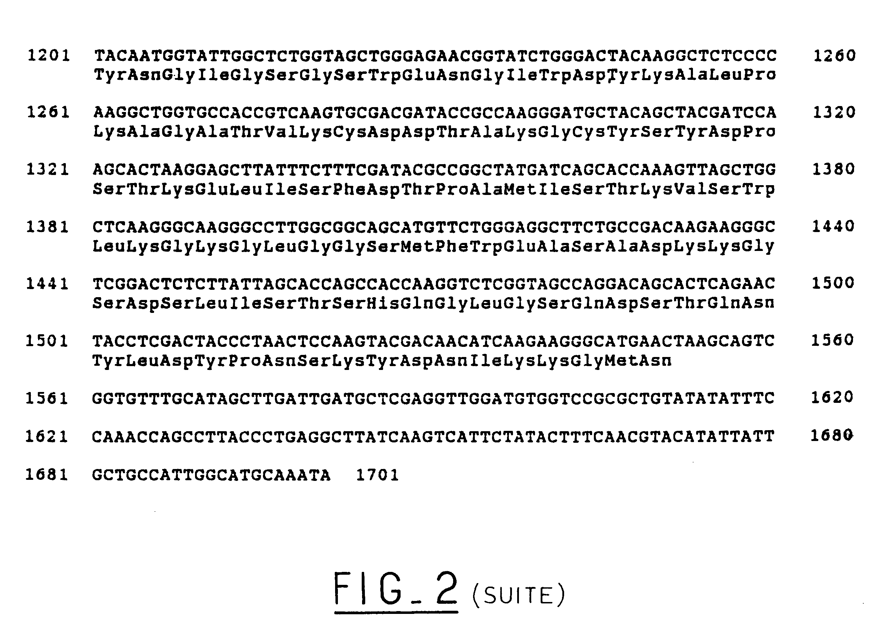

A partir de la séquence obtenue au moyen de ces premières amorces, d'autres amorces ont été déduites afin de pouvoir séquencer le reste du brin. En synthétisant des amorces dans le sens inverse, la séquence du brin complémentaire a été obtenue. Elle a permis de confirmer la séquence représentée dans la figure 2, qui sera commentée dans la section 7.

### Section 5 : Obtention d'un ADN complémentaire pleine longueur de la chitinase d'Aphanocladium album

### a) Constitution d'une banque d'ADN complémentaire

A partir des ARN messagers isolés comme décrit dans la section 2, on a réalisé une banque d'ADN complémentaire dans le vecteur pTZ19R (commercialisé par Pharmacia). Ce vecteur est un plasmide comprenant un polylinker contenant des sites uniques de restriction.

La technique de clonage utilisée est celle décrite par Caput et al., Proc. Ntl. Acad. Sci. (USA), (1986), 83, 1670-1674.

Elle consiste d'une part à digérer le vecteur par PstI, ajouter une queue de polydC sur l'extrémité 3' protubérante, puis à digérer le plasmide ainsi obtenu par BamHI. Le fragment correspondant au vecteur est purifié sur colonne de Sépharose CL4B (Pharmacia). Il comprend donc une queue polydC à une extrémité, l'autre extrémité étant cohésive, du type BamHI. D'autre part, les ARN messagers sont soumis à la transcription inverse à partir d'une amorce dont la séquence est la suivante (SEQ ID n° : 17) : 5'<GATCCGGGCCCT₍₁₂₎<3'. Ainsi, les ADNc présentent-ils à leur extrémité 5' la séquence GATC complémentaire de l'extrémité cohésive BamHI.

Les hybrides ARN-ADN obtenus par action de la transcriptase inverse sont soumis à une hydrolyse alcaline qui permet de se débarrasser de l'ARN. Les ADN complémentaires simple brin sont alors purifiés par 2 cycles sur colonne de Sépharose CL4 et soumis à un traitement à la terminal transférase de façon à ajouter des polydG en 3'. Les ADN complémentaires sont insérés sous forme simple brin dans le vecteur préparé comme décrit plus haut. Un second oligonucléotide appelé l'adaptateur ("adapter") complémentaire de l'amorce est nécessaire pour générer une extrémité cohésive BamHI à l'extrémité 5' des ADN complémentaires. Après hybridation du vecteur, de l'ADN complémentaire et de l'adaptateur, les molécules recombinantes sont circularisées par l'action de la ligase du phage T4. Les régions simple brin sont alors complétées en double brin grâce à l'ADN polymérase du phage T4.

Le pool de plasmides ainsi obtenu sert à transformer la souche MC 1061 (Casabadan, Chou et Cohen J. Bact. (1980), 143, 9971-9980) et les bactéries recombinantes sont sélectionnées pour la résistance à l'ampicilline.

### b) Criblage de la banque

### Préparation des sondes marquées :

Trois sondes déduites de la séquence du clone d'ADN complémentaire CH3C et du clone d'ADN génomique BL1 (section 4) ont été synthétisées à l'aide d'un synthétiseur d'ADN Biosearch 4600.

Les sondes sont les suivantes :

Elles codent respectivement pour des séquences situées au début, au milieu et en aval de la séquence codante de la chitinase d'Aphanocladium album. Les sondes sont marquées par couplage à la peroxydase.

### Hybridation et détection des colonies contenant l'ADN complémentaire de la chitinase d'Aphanocladium album :

Environ 100 000 colonies sont criblées par la technique d'hybridation in situ mise au point par Grunstein et Hogness (1975, Proc. Ntl. Acad. Sci. (USA), 72, 3961). Environ 10 000 bactéries sont étalées sur 10 boîtes de Pétri de façon à obtenir des colonies isolées. Après incubation pendant 24 h à 37°C, chaque boîte est répliquée sur 2 filtres de Nylon Hybond N⁺ (Amersham), chaque filtre étant destiné à être traité avec les trois sondes.

Les répliques sur membrane sont déposées avec les colonies vers le haut sur une feuille de Whatman 3MM saturée avec une solution de composition : NaOH 0,5 M ; NaCl 1,5 M, pendant 5 min, puis les membranes sont déposées sur une solution neutralisante de composition : NaCl 1,5 M ; Tris-HCl pH 8,0 0,5 M, pendant 5 min. Les répliques sont ensuite plongées dans une solution de 2 x SSC (NaCl 0,30 M, citrate de sodium 0,030M).

On traite ensuite les répliques sur membrane avec de la protéinase K (Boehringer Mannheim GmbH) à 100 µg/ml dans une solution de composition : Tris-HCl 10 mM pH 8 ; EDTA 10 mM ; NaCl 50 mM ; SDS 0,1 % à raison de 20 ml par membrane. On incube pendant 30 min à 37°C avec agitation. Les répliques sur membrane sont de nouveau plongées dans une solution de 2 x SSC et les débris bactériens sont partiellement éliminés en frottant doucement avec un papier de la marque Kim Wipes. Les membranes sont alors traitées pendant 5 min dans une solution de NaOH 0,4 M, puis rincées brièvement dans une solution de 2 x SSC. On obtient ainsi, pour chaque boîte, deux répliques sur membrane.

Les filtres sont mis à préhybrider dans un tampon contenant 0,1 % SDS, 6 x SSC, 10 x Denhardt et 100 µg/ml d'ADN de sperme de saumon soniqué et dénaturé (Sigma). La température de préhybridation est de 42°C et la durée de 6 h.

L'hybridation est réalisée à 42°C pendant 16 h en ajoutant 60 ng/ml du mélange des 3 sondes marquées à la peroxydase.

Le lavage des membranes est assuré dans la solution 2 x SSC ; 0,1 % SDS à 22°C pendant 2 fois 5 min, puis pendant 30 min, puis par 2 lavages de 15 min dans la solution 0,1 x SSC + 0,1 % SDS à 42°C et enfin 3 min dans une solution à 2 x SSC à 22°C.

La révélation se fait à l'aide du kit ECL d'Amersham (réf. RPN2110) selon le protocole du fabricant en utilisant les films Xomat AR (Kodak).

35 colonies présentaient une très forte hybridation avec le mélange des 3 sondes.

L'ADN plasmidique de 15 de ces 35 colonies a été préparé et digéré par les enzymes HindIII et BamHI. Après électrophorèse sur gel d'agarose à 1 %, les doubles digestions permettent de libérer un fragment dont la taille est d'environ 1,6 kb dans 4 cas sur les 15.

### Section 6 : Détermination de la séquence de l'ADN complémentaire pleine longueur de la chitinase d'Aphanocladium album

On a recloné un des fragments de 1,6 kb dans l'ADN de la forme réplicative du phage M13. L'ADN des clones M13 contenant le fragment de 1,6 kb a été soumis à une digestion exonucléasique de manière à générer une série de clones M13 chevauchants (procédure "Cyclone I Biosystem" de IBI). Ces derniers ont été séquencés par la méthode des didéoxyribonucléotides (Sanger et al., 1977, Proc. Ntl. Acad. Sci. USA, 14, 5463-5467).

### a) Analyse de la séquence de l'ADNc de la chitinase d'Aphanocladium album

La description ci-après sera mieux comprise à l'aide de la figure 1. Cette séquence renferme une seule phase de lecture ouverte (non interrompue par un codon stop) compatible avec le poids moléculaire apparent de la protéine observé par électrophorèse sur gel de polyacrylamide 12,5 % : la séquence commençant par un codon ATG en position 97 et terminée par le codon stop TAA en position 1366, codant pour une protéine de 423 acides aminés et de poids moléculaire d'environ 46 kDa.

Un peptide signal est attendu par l'homme de l'art car les chitinases sont des protéines qui peuvent être naturellement sécrétées par les cellules de champignon, ce qui exige la présence d'un peptide signal.

Le logiciel UWGCG de l'Université du Wisconsin : Devereux et al., 1984, Nucl. Ac. Res., 12, 8711-8721 - option Recherche de peptide signal d'après la méthode de G. Von Heijne, 1986, Nucl. Ac. Res., 14, 483-490, prévoit dans cette séquence une partie codant pour un peptide signal reconnu par les cellules eucaryotes ou procaryotes, la séquence (Na2) suivante (SEQ ID n° : 7), appelée séquence nucléotidique pré (commençant au nucléotide 97 et se terminant au nucléotide 162) : codant pour le peptide signal de 22 acides aminés de séquence (a2) suivante (SEQ ID n° : 4) :

Entre la séquence codant pour le peptide signal ci-dessus et celle codant pour la protéine mature, il y a la séquence nucléotidique (Na3) suivante (SEQ ID n° : 8), appelée séquence nucléotidique pro, commençant au nucléotide 163 et se terminant au nucléotide 198. codant pour la séquence peptidique (a3) suivante (SEQ ID n° 5), appelée séquence peptidique pro Il y a donc, en amont de la séquence codant pour la chitinase d'Aphanocladium album mature la séquence codant pour la séquence peptidique prépro (a4) suivante (SEQ ID n° : 21) :

La protéine mature est la protéine de 389 acides aminés d'un poids moléculaire voisin de 42,8 kDa qui commence par la séquence aminoterminale déterminée dans la section 1. Le poids moléculaire apparent observé d'environ 41 ± 3 kDa correspond, compte tenu de la marge d'erreur expérimentale, au poids moléculaire de 42,8 kDa calculé de la protéine déduite de l'ADN complémentaire. Cette protéine possède deux sites potentiels de N-glycosylation (soulignés sur la figure 1).

### Comparaison de la séquence peptidique (a1) aux autres séquences peptidiques déjà connues

La comparaison a porté sur les chitinases végétales de classes I, II et III, définies par Shinshi et al., 1990, Plant. Mol. Biol., 14, 357-368 et sur les chitinases bactériennes de Bacillus circulans, Serratia marcescens et Streptomyces erythraeus. La comparaison a été effectuée à l'aide du logiciel UWGCG de l'Université du Wisconsin : Devereux et al., 1984, Nucl. Ac., Res. 12, 8711-8721, option GAP. Cette méthode algorithmique considère tous les alignements possibles et crée un alignement dans lequel le plus possible d'acides aminés identiques sont appariés et le nombre de trous dans les séquences alignées est minimal.

La séquence peptidique la plus proche de la séquence (a1) déduite de l'ADN complémentaire d'A. album est celle de la chitinase de Serratia marcescens (Jones J.D.G. et al., 1986, EMBO J., 5, 467-473) avec une homologie d'environ 33 %. Cette dernière chitinase est une exochitinase (Kuntz, 1991, Thèse de Doctorat - Université P. et M. Curie, Paris).

### Section 7 : Analyse de la séquence de l'ADN génomique de la chitinase d'Aphanocladium album

La description ci-après sera mieux comprise à l'aide de la figure 2. L'alignement de cette séquence avec la séquence d'ADNc de la figure 1 montre que ce gène code pour la même séquence peptidique que l'ADN complémentaire cloné et met en évidence trois introns de petite taille. La séquence commence par un codon ATG en position 126 et se termine par un codon stop TAA en position 1552. Le premier intron commence en position 266 et se termine en position 320. Le deuxième intron commence en position 420 et se termine en position 472. Le troisième intron commence en position 523 et se termine en position 571. Cette séquence code pour une protéine de 423 acides aminés.

### Section 8 : Expression de la chitinase d'Aphanocladium album dans Fusarium oxysporum

La cotransformation, qui repose sur la complémentation d'une souche réceptrice du champignon filamenteux Fusarium oxysporum f. sp. melonis de phénotype nitrate réductase moins (nia-), est effectuée par le mélange d'un plasmide portant un gène codant pour une nitrate réductase d'Aspergillus nidulans et du plasmide pBL1 défini précédemment, porteur de-l'ADN génomique de la chitinase d'Aphanocladium album. La sélection des transformants s'opère sur un milieu contenant une source de nitrate permettant aux seuls transformants ayant intégré le gène de la nitrate réductase de survivre et de se développer. Certains de ces transformants ont également intégré le fragment fBL1. C'est parmi ces doubles transformants que sont recherchés ceux qui expriment une activité chitinase.

### a) Transformation de Fusarium oxysporum

La souche réceptrice de F. oxysporum f. sp. melonis est la souche Fom150 nia9 décrite par Daboussi et al., 1989, Curr. Genet., 15, 453-456.

Le gène niad codant pour la nitrate réductase d'Aspergillus nidulans porté sur un fragment Sau3A de 11 kb est inséré dans le plasmide pFB39 qui est dérivé de pUC19. Ce plasmide utilisé pour la transformation de F. oxysporum porte le nom de pAN301. Sa construction est décrite par Malardier et al., 1984, Gene 78 : 147-156.

La cotransformation est réalisée en utilisant un mélange de plasmide pAN301 et de plasmide pBL1 décrit précédemment dans un rapport 1/1. Les conditions expérimentales utilisées pour assurer la transformation des protoplastes de F. oxysporum, et la sélection des transformants sur nitrate sont celles décrites par LANGIN T. et al., (1990), Curr. Genet., 17 : 313-319.

On a ainsi obtenu quatre cotransformants, appelés Tr5, Tr6, Tr7 et Tr8.

### b) Expression de la chitinase d'Aphanocladium album par les cotransformants de Fusarium oxysporum

Une analyse selon la méthode de Western blot a été effectuée sur ces 4 cotransformants de la façon décrite ci-après. Les protéines du milieu de culture sont concentrées contre du polyéthylèneglycol (Carbowax 20M-Touzart et Matignon) et l'extrait est ensuite dialysé contre un tampon acétate de sodium 0,2 M pH 5,0, puis dilué dans un tampon de charge de composition suivante :
- 0,125 M Tris-HCl pH 6,8
- 4 % dodécylsulfate de sodium
- 20 % glycérol
- 0,002 % bleu de bromophénol
- 10 % β-mercaptoéthanol
puis le mélange est porté à 100°C pendant 10 min. 10 µg de protéines solubilisées sont déposés sur un gel d'électrophorèse de SDS polyacrylamide selon le protocole décrit par Laemmli (Laemmli, Nature, 227, 1970, 680-685). Après électrophorèse, les protéines du gel sont transférées sur une membrane Immobilon (en PVDF) par électrotransfert selon la technique de H. Towbin et al., Proc. Natl. Acad. Sci. USA, 76, 1979, 4350-4354.

L'immunodétection est réalisée selon un protocole qui comprend les étapes suivantes :
- saturation de la membrane PVDF sur laquelle les protéines ont été transférées par incubation pendant au minimum 2 h à 37°C dans une solution de gélatine à 3 %,
- 3 lavages dans du tampon phosphate salin contenant 0,05 % de détergent Tween 20,
- incubation (pendant 1 h à 37°C) en présence de l'immunsérum préparé précédemment (contenant les anticorps polyclonaux reconnaissant la chitinase d'Aphanocladium album) dilué au 1/10 000^{e} dans du tampon phosphate salin,
- 3 lavages dans du tampon phosphate salin contenant 0,05 % de détergent Tween 20.

Le complexe antigène-anticorps est ensuite révélé à l'aide d'un système streptavidine-biotine conjugé à la phosphatase alcaline avec le kit RPN 23 d'Amersham ("Blotting détection kit"), utilisé selon les indications du fabricant.

L'empreinte obtenue montre pour les cotransformants Tr8 et Tr7 la présence d'une protéine d'environ 41 + 3 kDa de même poids moléculaire apparent que la chitinase d'A. album purifiée obtenue dans la section 1, absente pour la souche témoin.

La non-expression pour les cotransformants Tr5 et Tr6 résulte probablement de l'insertion du gène recombinant dans un contexte ne permettant pas la transcription.

L'activité chitinase de la souche F. oxysporum sauvage (témoin) et celles des cotransformants Tr7 et Tr8 sont mesurées par la méthode radiochimique de Molano et al. (1977, Anal. Biochem., 83, 648-656), résumée au paragraphe (b) de la section 1. La quantité totale de protéines est déterminée par la méthode de Bradford (Bradford M.M., 1976, Anal. Biochem., 72, 248-254). Les résultats sont rassemblés sur le tableau (I) ci-après. On constate que le milieu de culture du cotransformant Tr8, qui exprime le mieux la chitinase d'A. album, voit son activité chitinolytique spécifique augmenter d'un facteur de 25 à 30 par comparaison avec la souche témoin. De plus, l'activité de la chitinase extracellulaire des cotransformants Tr8 et Tr7 diminue lorsqu'on ajoute les anticorps antichitinase d'Aphanocladium album (préparés dans la section 1).

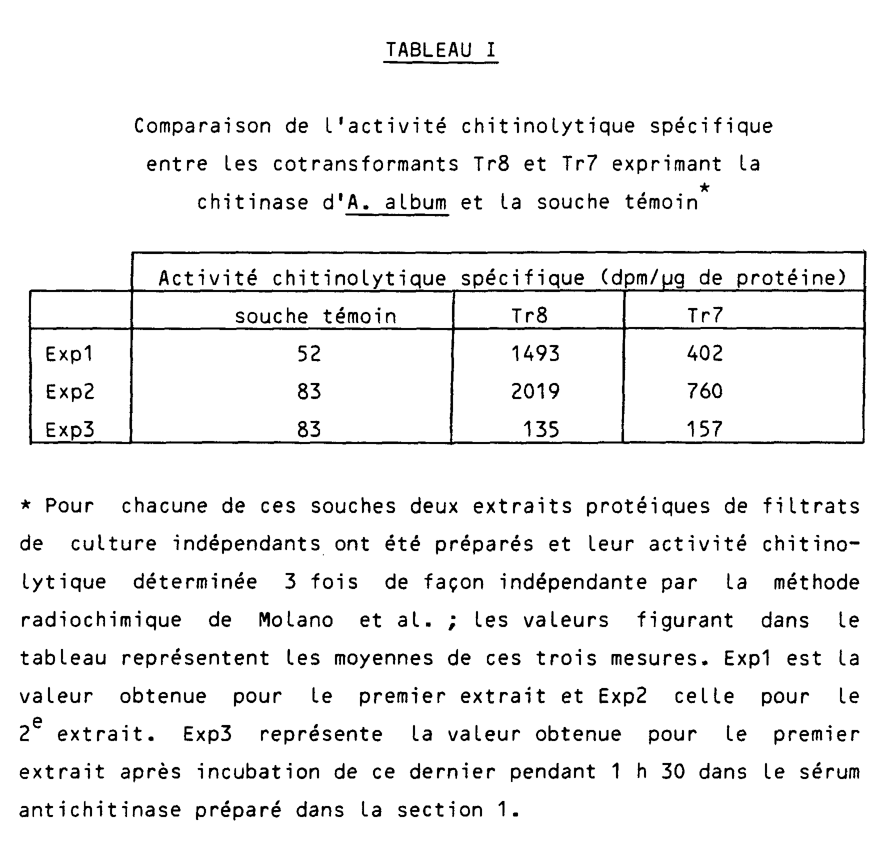

### Section 9 : Expression de la chitinase d'Aphanocladium album mature dans E. coli

### a) Construction du plasmide intermédiaire pEMR680

Le plasmide pUC18 (Clontech) qui comprend un polylinker portant notamment les sites PstI, SalI et BamHI est linéarisé par coupure à l'aide des endonucléases PstI et SalI. Le fragment PstI-SalI portant l'origine de réplication est purifié.

A partir de l'ADNc (cf. section 6 et figure 1), par coupure à l'aide des enzymes de restriction PstI et XhoI, on génère un fragment PstI-XhoI d'environ 1 250 paires de bases. Ce fragment a été purifié sur gel d'agarose, puis ligué à l'aide de la polymérase T4 au fragment PstI-SalI précédent (les sites SalI et XhoI disparaissent par ligation). Le mélange de ligation sert à transformer la souche E. coli RRI.

Le plasmide obtenu est appelé plasmide pEMR680. Ce plasmide renferme un fragment incomplet dans sa partie 5' de l'ADNc codant pour la préprochitinase d'Aphanocladium album. Il manque 47 paires de bases entre le site PstI et le codon ATG marquant le début de la traduction.

### b) Construction du vecteur d'expression dans E. coli : le plasmide pEMR 682

L'hydrolyse partielle du plasmide pEMR680 par l'enzyme AccI et totale par l'enzyme BamHI permet de libérer un fragment d'environ 1100 pb. Ce fragment code pour une partie 3' de la séquence codante de la chitinase d'A. album mature (cf. figure 1).

L'oligonucléotide de synthèse de séquence (Nt1) suivante (SEQ ID n^{o} : 22) : permet de reconstituer l'extrémité 5' de la chitinase d'A. album mature. Le premier acide aminé de la séquence de la protéine mature (codé par GGT) est précédé par l'extrémité cohésive d'un site NdeI portant un codon ATG initiateur de la traduction.

Le plasmide pAR3040 (Studier F.U. et al., 1986, J. Mol. Biol., 189, 113-130) porte un fragment de pBR322 comprenant l'origine de réplication dans E. coli et dans le gène codant pour la résistance à l'ampicilline, un fragment d'ADN portant le gène 10 du phage T7 ainsi que son promoteur et son terminateur. Le gène 10 peut être facilement remplacé par tout autre gène d'intérêt puisqu'il est borné par les séquences reconnues par les enzymes de restriction NdeI et BamHI.

Le plasmide pAR3040 est hydrolysé par les enzymes NdeI et BamHI et le fragment portant l'origine de réplication dans E. coli, le gène de résistance à l'ampicilline ainsi que le promoteur et le terminateur du gène 10 du phage T7 est purifié. L'assemblage par ligation de l'oligonucléotide de séquence (Nt1), du fragment de 1100 pb du plasmide pEMR680 et du fragment du plasmide pAR3040 conduit, après transformation du mélange de ligation dans la souche d'E. coli RRI à un plasmide appelé plasmide pEMR682.

### c) Expression de la chitinase mature dans E. coli

Le promoteur du gène 10 du phage T7 n'est pas reconnu par l'ARN polymérase d'E. coli. Il convient donc d'apporter à la souche E. coli RRI portant le plasmide pEMR682, appelée souche RRI/ pEMR682, une ARN polymérase qui reconnaît le promoteur du gène 10 du phage T7. Le moyen qui est choisi pour effectuer cette opération est d'utiliser le phage recombinant CE6 (Studier et al., référence ci-dessus) qui porte dans son génome le gène codant pour l'ARN polymérase du phage T7.

### - Constitution d'un stock de phages CE6

Cette expérience, bien connue de l'homme de l'art, est décrite dans Sambrook et al., op cité. Elle consiste à infecter 100 µl de la bactérie ED 8739 cultivée dans un milieu contenant 5 g d'extrait de levure, 10 g de bactotryptone, 5 g de NaCl, 10 mM MgSO₄ et 0,4 % de maltose par le phage CE6 de manière que la suspension soit composée de 1 phage pour 6 bactéries. Cette suspension est mélangée à 3 ml d'un milieu appelé Top Agar dont la composition est la même que celle indiquée ci-dessus, mais complémentée par 7 g/l d'agar. Le mélange maintenu à une température de 50°C est étalé sur une boîte de Pétri contenant le même milieu, mais complémenté par 15 g/l d'agar. Après une nuit d'incubation à une température de 37°C, la gélose est recouverte de plages de lyse contenant les phages. Les phages sont élués en raclant le Top Agar et en le mélangeant avec 3 ml de MgSO₄ 10 mM par boîte de Pétri pendant 1 h à température ambiante. Le mélange est ensuite centrifugé pendant 10 min à 15 000 g et le surnageant, qui contient les phages à une concentration de 2.10¹⁰ phages par ml de surnageant, est appelé stock de phages. Le stock de phages est conservé à une température de 4°C.

### - Expression de la chitinase

La souche RRI/pEMR682 est cultivée dans un milieu de composition identique à celle du milieu utilisé pour faire le stock de phages, mais sans agar. Après une incubation sous agitation à une température de 37°C cette culture est diluée 50 fois dans le même milieu et incubée à une température de 37°C afin d'obtenir une densité cellulaire de 10⁹ cellules par ml (DO = 1). Les phages sont ajoutés à la culture de telle manière que le mélange soit composé de 10 phages pour une bactérie. Le mélange est incubé pendant 2 h à une température de 42°C. Le culot bactérien est ensuite obtenu par centrifugation pendant 10 min à 15 000 g.

### d) Analyse de la chitinase produite

### - Gel de polyacrylamide

Un culot bactérien issu d'une culture de 1 ml ayant atteint une densité optique mesurée à 600 nm de 0,2 est mis en suspension dans un tampon dont la composition est la suivante : 0,125 mM de Tris HCl pH 6,8, 4 % dodécylsulfate de sodium, 20 % glycérol, 0,002 % bleu de bromophénol et 10 % β-mercaptoéthanol. Le mélange est immergé pendant 10 min dans de l'eau bouillante, ce qui permet la lyse des bactéries et la solubilisation des protéines. Le mélange est ensuite soumis à une électrophorèse sur gel de polyacrylamide 12,5 % (Laemmli UK, 1970, Nature, 227, 680-685) en présence d'un marqueur de taille et de la chitinase d'A. album purifiée obtenue dans la section 1. Après décoloration du gel, préalablement coloré au bleu de Coomassie, on constate qu'il existe pour la souche RRI/pEMR682 une bande supplémentaire représentant approximativement 20 % de protéines totales par rapport à la souche RRI/pBR322 mise en témoin et traitée dans les mêmes conditions. Cette bande présente un poids moléculaire apparent de l'ordre de 39 + 3 kDa, légèrement inférieur au poids moléculaire apparent de la chitinase d'A. album purifiée, obtenue dans la section 1.

### - Immunoempreinte

Les conditions d'électrophorèse décrites au paragraphe précédent sont maintenues. Le gel n'est pas coloré au bleu de Coomassie, mais les protéines sont transférées sur un filtre de nitrocellulose selon la technique décrite par Towbin et al., 1979, Proc. Ntl. Acad. Sci-USA, 76, 4350-4354 et l'immunodétection est réalisée selon le protocole décrit dans la section 8.

L'empreinte obtenue montre que la protéine supplémentaire a un poids moléculaire apparent d'environ 39 + 3 kDa, légèrement inférieur à celui de la chitinase d'A. album, isolée dans la section 1. Cette protéine présente dans la souche RRI/pEMR682 et absente dans la souche témoin est reconnue par les anticorps dirigés contre la chitinase d'A. album.

### - Mise en évidence d'une activité chitinolytique dans E. coli

L'activité chitinolytique des extraits de la souche RRI/ pEMR682 et celle de la souche témoin est mesurée par la méthode radiochimique de Molano et al. résumée au paragraphe b1 de la section 1. Les résultats, rassemblés dans le tableau II ci-dessous, montrent que la souche RRI/pEMR682 présente, contrairement à la souche témoin, une activité chitinolytique.

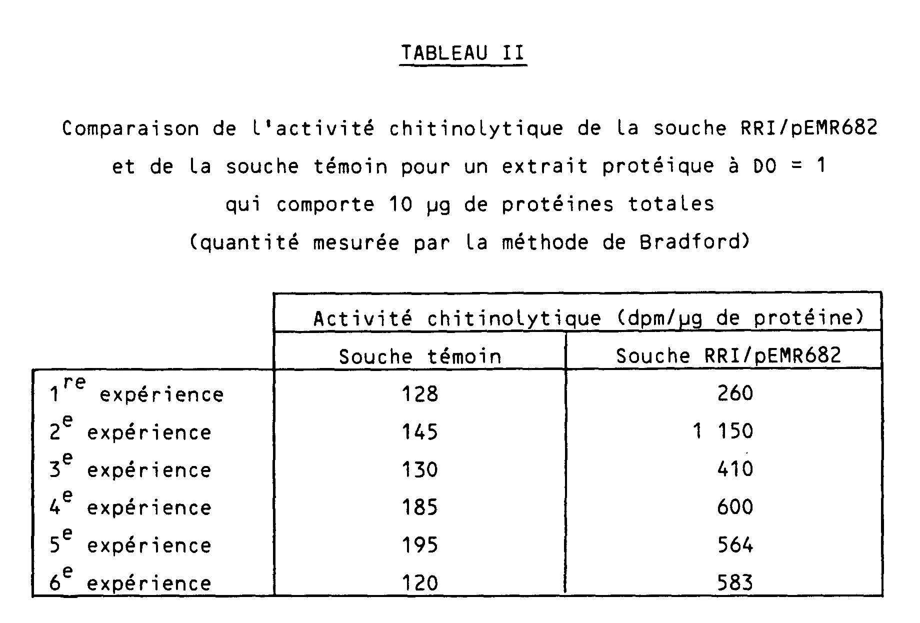

### Section 10 : Expression de la chitinase d'Aphanocladium album dans la levure (Saccharomyces cerevisiae) et sécrétion à l'aide du peptide signal de la phéromone α

### a) Construction du plasmide pEMR698, vecteur d'expression de la chitinase d'Aphanocladium album mature :

Le plasmide pEMR583 est décrit dans le document EP-0 273 800. Il comprend notamment un fragment du plasmide pBR322 portant l'origine de réplication dans E. coli et le gène de résistance à l'ampicilline, un fragment du plasmide 2 µ qui permet la réplication dans la levure, les gènes complémentant l'auxotrophie de la leucine, ainsi qu'une cassette d'expression de l'IL-8 comportant un promoteur hybride Gal7-ADH₂, le peptide signal de la phéromone α, une séquence codant pour l'IL-8 mature et un terminateur.

Le plasmide pEMR583 est hydrolysé par les enzymes HindIII et BamHI et le grand fragment HindIII-BamHI est purifié ; ce fragment porte le système d'expression nécessaire dans la levure. Il est assemblé et ligué avec le fragment AccI-BamHI issu du plasmide pEMR680 décrit à la section 9 et avec un oligonucléotide synthétique HindIII-AccI de séquence (Nt2) suivante et qui reconstitue la séquence 3' de la région prépro de la phéromone α de S. cerevisiae manquant au grand fragment HindIII-BamHI issu du plasmide pEMR583 et l'extrémité 5' de la chitinase mature d'A. album manquant au fragment AccI-BamHI. Le mélange de ligation a servi à transformer une souche E. coli RRI. Le plasmide pEMR698 est obtenu.

### b) Transformation de la souche de levure EMY761 par le plasmide pEMR698 avec sélection pour la prototrophie de leucine

La souche EMY761 est une souche de levure Matα , leu2, ura3, his3 et gal qui dérive de la souche GRF18 bien connue de l'homme de l'art (Gerry Fink, MIT, USA) par croisements successifs de cette souche avec une souche ura3 obtenue à partir de la souche FL100 (déposée à l'ATCC sous le n° 28 383) et avec la souche 20B12 (Matα, trp1, pep4) décrite par E.W. Jones, 1987, Genetics, 85, 23. Cette souche peut s'obtenir par curage plasmidique de la souche déposée à la CNCM le 27.12.1990, sous le n° I 1022.

La technique de transformation utilisée est une variante de celle décrite par Beggs et al. (Beggs et al., (1978), Nature, 275, 104-109). Elle consiste à soumettre les levures à un traitement de protoplastisation en présence d'un stabilisant osmotique, le sorbitol en concentration 1 M.

Le protocole précis de transformation est précisé ci-après :
a) 200 ml du milieu YPG liquide (cf. tableau III ci-après) sont inoculés avec environ 5 x 10⁶ cellules d'une culture en phase stationnaire et la culture ainsi inoculée est placée une nuit sous agitation à 30°C.
b) Lorsque la culture atteint environ 10⁷ cellules par millilitre, les cellules sont centrifugées à 4 000 tr/min pendant 5 min et le culot est lavé avec du sorbitol 1 M.
c) Les cellules sont suspendues dans 5 ml de solution de sorbitol 1 M contenant 25 mM EDTA et 50 mM de dithiothréitol et incubées pendant 10 min à 30°C.
d) Les cellules sont lavées une fois avec 10 ml de sorbitol 1 M et suspendues dans 20 ml de sorbitol. De la zymolyase-100T (préparation obtenue par purification partielle sur colonne d'affinité du surnageant de culture d'Arthobacter luteus et contenant de la β-1,3-glucane-laminaripentahydrolase, commercialisée par SEYKAGAKU KOGYO Co. Ltd) est ajoutée à une concentration finale de 20 µg/ml et on incube la suspension à température ambiante pendant environ 15 min.
e) Les cellules sont resuspendues dans 20 ml d'un milieu contenant du sorbitol appelé milieu YPG sorbitol (cf. tableau III ci-après) et incubées pendant 20 min à 30°C sous agitation douce.
f) On centrifuge pendant 3 min à 2 500 tr/min.
g) On resuspend dans 9 ml de tampon de transformation (sorbitol 1 M, tris-HCl de pH 7,5 10 mM et CaCl₂ 10 mM).
h) On ajoute 0,1 ml de cellules et 5 µl de solution d'ADN (environ 5 µg) du plasmide pEMR698 et on laisse la suspension obtenue pendant 10 à 15 min à température ambiante.
i) On ajoute 1 ml de la solution : polyéthylèneglycol PEG 4000 20 %, tris-HCl de pH 7,5 10 mM et CaCl₂ 10 mM.
j) On verse 0,1 ml de la suspension obtenue en i) dans un tube contenant du milieu solide de régénération sans leucine (cf. tableau III ci-après) préalablement fondu et maintenu liquide à environ 45°C. On verse la suspension sur une boîte de Pétri contenant une couche solidifiée de 15 ml de milieu de régénération solide sans leucine.
k) On répète l'étape j) avec le reste de la suspension cellulaire obtenue en i).

Les transformants commencent à apparaître au bout de 3 jours.

Le transformant retenu est appelé EMY761/pEMR698

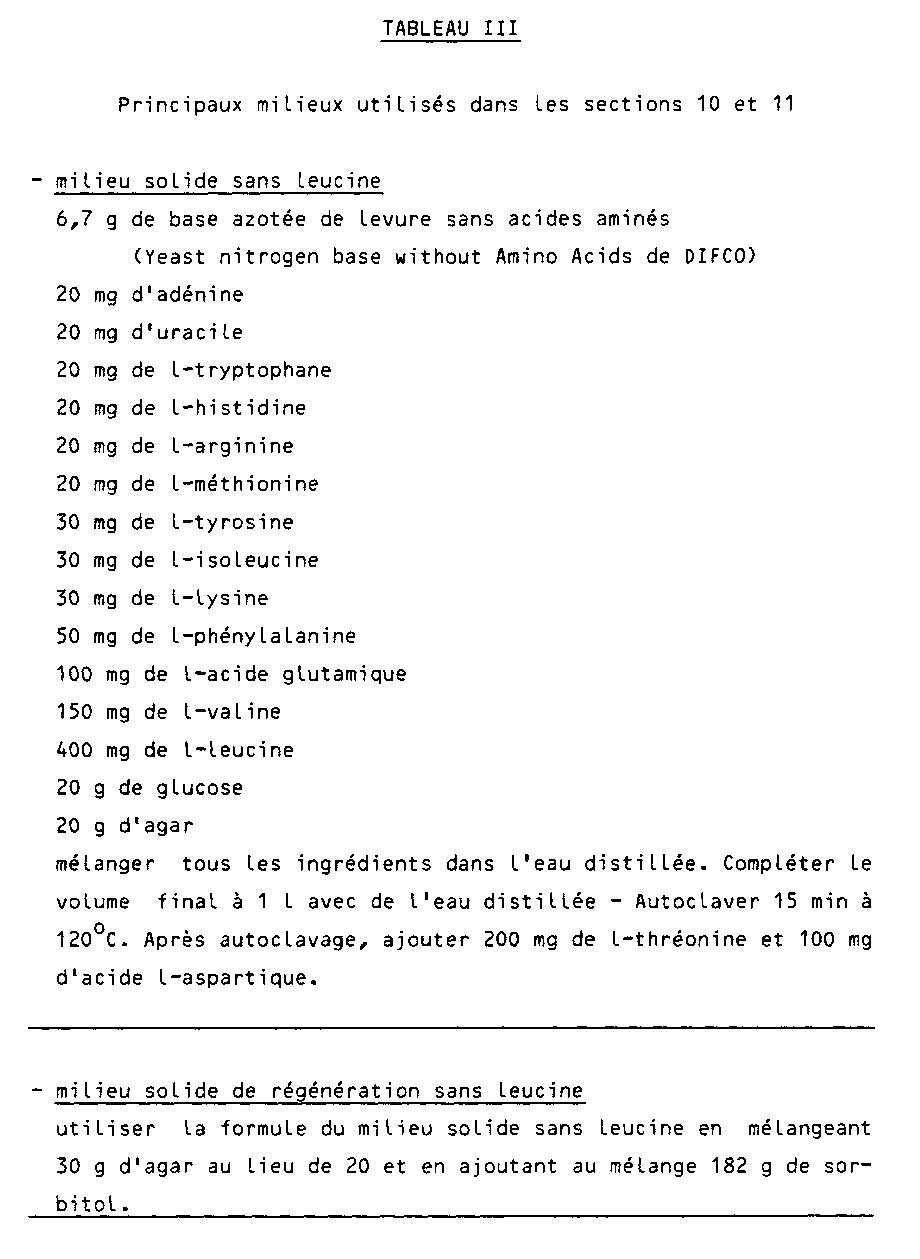

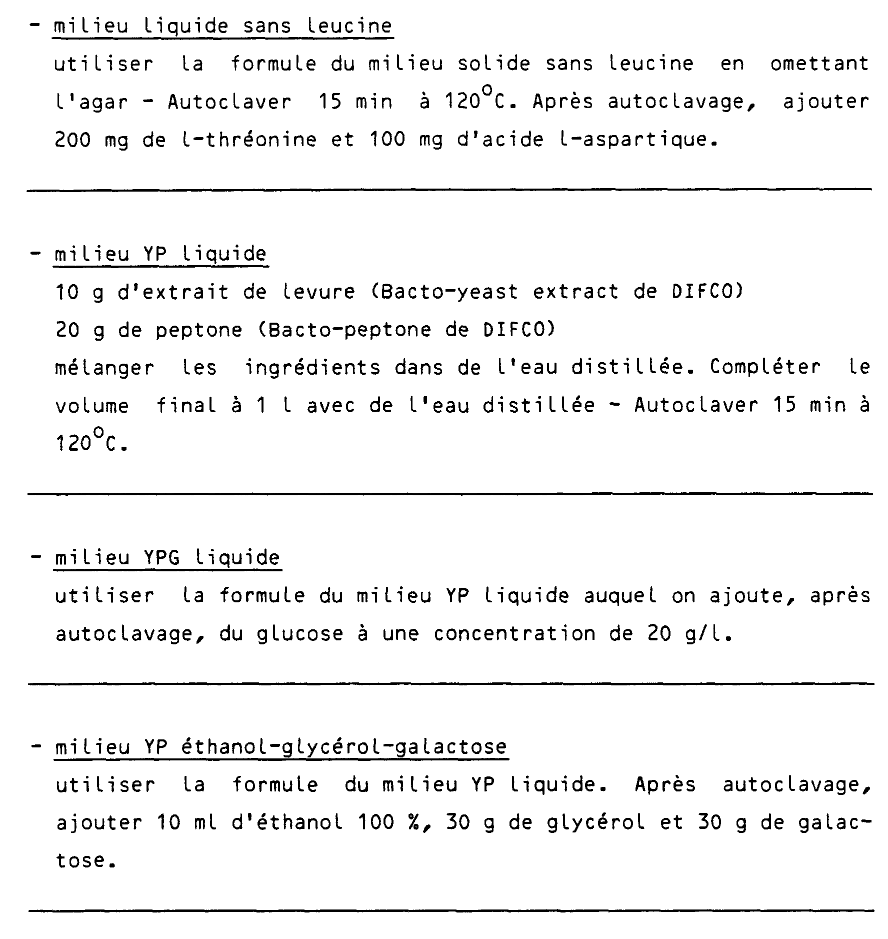

### c) Expression de la chitinase d'A. album par la souche EMY761/ pEMR698

Les précultures se font dans du milieu de composition : milieu YP liquide 1,4 %, glucose 3 %, histidine 50 µg/ml et uracile 50 µg/ml. Au bout de 24 h, on centrifuge les cultures et on reprend le culot dans 40 ml de milieu de composition : milieu YP liquide 1,4 %, éthanol 1 %, casaminoacides 1 %, uracile 100 % µg/ml, glycérol 3 % et galactose 1 %.

### d) Analyse des protéines présentes dans le milieu de culture

### - préparation des échantillons

Au bout de 24 h de culture, les cellules cultivées en c) ont été centrifugées pendant 20 min et le surnageant a été recueilli. A 10 ml de surnageant, 5 ml d'acide trichloroacétique à 50 % contenant 2 mg/ml de déoxycholate ont été rajoutés.

Le mélange a été mis à +4°C pendant 30 min, puis centrifugé pendant 30 min. Le culot a été repris dans environ 1 ml d'acétone froid (+4°C) et de nouveau centrifugé 30 min. Le culot, après avoir été séché, est repris dans environ 20 µl d'un tampon dénommé tampon de charge constitué de Tris-HCl 0,125 M pH 6,8 SDS 4 %, bleu de bromophénol 0,002 %, glycérol 20 %, β-mercaptoéthanol 10 % (selon protocole décrit par Laemmli (1970)). Le culot est solubilisé par ébullition pendant 15 min, puis neutralisé en ajoutant de la soude 10 N.

L'analyse des protéines par électrophorèse en gel SDS dénaturant est réalisée selon la méthode décrite dans la section 9d).

Le profil obtenu montre la présence d'une bande large surnuméraire présente dans la souche EMY761/pEMR698 et absente de la souche témoin (souche EMY761 non transformée). Cette bande a un poids moléculaire compris entre 39 et 46 kDa. La largeur de cette bande résulte probablement de différents degrés de glycosylation de la protéine, qui possède (cf. figure 1) deux sites de N-glycosylation potentiels. On montre par Western blot que cette protéine est reconnue par les anticorps dirigés contre la chitinase d'Aphanocladium album.

### e) Mise en évidence d'une activité chitinolytique dans le surnageant de culture de Saccharomyces cerevisiae

L'activité chitinolytique des extraits de surnageant de culture de la souche EMY761/pEMR698 et de la souche témoin est mesurée par la méthode radiochimique de Molano (1977, Anal. Biochem, 83, 648-656), résumée au paragraphe b1 de la section 1.

Les résultats, rassemblés dans le tableau IV ci-après, montrent que l'activité chitinolytique du surnageant de culture de la souche transformée par le plasmide pEMR698 est supérieure à celle du surnageant de culture enregistrée dans la souche témoin.

**TABLEAU IV**

| Comparaison de l'activité chitinolytique de la souche de levure EMY761/pEMR698 et de la souche témoin, pour un extrait de surnageant de culture concentré par diafiltration qui comporte 10µg de protéines totales (quantité mesurée selon la méthode de Bradford). Chaque valeur est la moyenne de 4 mesures indépendantes. | | |
|---|---|---|
| | ACTIVITE CHITINOLYTIQUE (dpm/µg de protéine) | |
| | Souche témoin | Souche EMY761/pEMR698 |
| expérience 1 | 156 | 730 |
| expérience 2 | 270 | 560 |
| expérience 3 | 120 | 750 |

### Section 11 : Expression de la chitinase d'A. album dans la levure et sécrétion à l'aide de son propre peptide signal

### a) Construction du plasmide pEMR697, vecteur d'expression de la préprochitinase d'Aphanocladium album

Le plasmide pEMR473 est décrit dans la demande de brevet EP-0 408 461. Il comprend notamment un fragment du plasmide pBR322 portant l'origine de réplication dans E. coli et le gène de résistance à l'ampicilline, un fragment du plasmide 2 µ qui permet la réplication dans la levure, les gènes complémentant l'auxotrophie de la leucine, ainsi qu'une cassette d'expression de l'urate oxydase d'Aspergillus flavus comportant un promoteur hybride Gal7-ADH₂, une séquence codant pour l'urate oxydase d'A. flavus et un terminateur.

Le plasmide pEMR473 est hydrolysé partiellement par ClaI et complètement par BamHI. Le grand fragment BamHI-ClaI purifié est le plasmide entier délété du gène codant pour l'urate oxydase. Ce fragment est ligué en présence de l'oligonucléotide synthétique de séquence (Nt3) ce qui permet l'association entre l'extrémité 3' non codante de l'ADN complémentaire de l'urate oxydase, la partie 5' de la séquence prépro de la chitinase d'A. album et le fragment PstI-BamHI du pEMR680 qui porte la partie 3' de la séquence codante de la chitinase d'A. album. Le mélange de ligation est utilisé pour transformer E. coli RRI. Le plasmide résultant est le pEMR697.

### b) Transformation de la souche de levure EMY761 par le plasmide pEMR697 avec sélection pour la prototrophie de leucine.

cf. Section 10 b)
Le transformant est appelé EMY761/pEMR697

### c) Culture de la souche EMY761/pEMR697

cf. Section 10 c)

### d) Analyse des protéines présentes dans le milieu de culture

cf. Section 10 d)

### e) Mise en évidence d'une activité chitinolytique dans Saccharomyces cerevisiae

cf. Section 10 e)

Les résultats sont rassemblés dans le tableau V ci-après.

**TABLEAU V**

| Comparaison de l'activité chitinolytique de la souche de levure EMY761/pEMR697 et de la souche témoin pour un extrait de surnageant de culture qui comporte 10 µg de protéines totales (quantité mesurée selon la méthode de Bradford) | | |
|---|---|---|
| | Activité chitinolytique (dpm/µg de protéine) | |
| | Souche témoin | Souche EMY761/pEMR697 |
| expérience 1 | 156 | 624 |
| expérience 2 | 270 | 2 819 |
| expérience 3 | 120 | 350 |

### Section 12 : Construction de deux vecteurs d'expression de la chitinase d'Aphanocladium album dans les cellules végétales

### a) Préparation d'un gène codant pour la chitinase d'A. album mature précédée d'un peptide signal

### Construction 1 : gène codant pour la préprochitinase d'A. album.

L'ADN complémentaire portant la séquence codante de la préprochitinase d'A. album a été obtenu par coupure du vecteur pEMR697, décrit dans la section 11, à l'aide des enzymes de restriction ClaI et BamHI. Le fragment obtenu a été purifié par électrophorèse sur gel d'agarose à bas point de fusion. Ce fragment porte la séquence codante de l'ADN complémentaire de la préprochitinase d'Aphanocladium album, l'ATG initiateur étant précédé d'un fragment de 11 pb portant le site ClaI. Ce fragment a été ligué d'une part en 5' avec l'oligonucléotide de séquence (Nt4) ci-après, porteur en 5' d'un site BamHI et en 3' d'un site ClaI, afin de reconstituer un site BamHI, et d'autre part en 3', avec l'oligonucléotide de séquence (Nt5) ci-après porteur en 5' d'un site cohésif compatible avec le site BamHI et en 3' d'un site SacI, afin de reconstituer un site SacI. Le fragment obtenu contient la séquence codante de l'ADN complémentaire de la préprochitinase d'Aphanocladium album entouré par un site BamHI en 5' et un site SacI en 3'. Ces deux sites permettront ultérieurement une insertion avec un promoteur et un terminateur.

La séquence des oligonucléotides utilisés est fournie ci-après, les sites de restriction étant indiqués entre parenthèses : La ligation est réalisée à l'aide de l'ADN ligase T4.

### Construction 2 : Gène codant pour la chitinase d'Aphanocladium album mature précédée du peptide signal d'une chitinase de haricot

L'ADN complémentaire portant une partie de la séquence codante de la chitinase d'Aphanocladium album mature a été obtenu à partir du plasmide pEMR680 décrit dans la section 9 par digestion partielle à l'aide des endonucléases AccI et BamHI. Le fragment obtenu d'environ 1 100 paires de bases, qui contient la partie codante de la chitinase d'A. album mature à l'exception des 23 premières paires de bases, est ligué d'une part en 5' avec l'oligonucléotide de séquence (Nt6) ci-après et, d'autre part, en 3', avec l'oligonucléotide de séquence (Nt5). L'oligonucléotide (Nt6) contient en 5' un site BamHI, la séquence codant pour le peptide signal d'une chitinase de haricot (Broglie K.E. et al., 1986, Proc. Ntl. Acad. Sci. USA, 83, 6820-6824), les 23 premières paires de bases manquantes de la chitinase mature d'A. album et un site AccI en 3'.

Le fragment obtenu contient la séquence codante de la chitinase d'Aphanocladium album mature fusionnée à la séquence codante du peptide signal d'une chitinase de haricot, entourée d'un site BamHI en 5' et d'un site SacI en 3'.

La séquence de l'oligonucléotide utilisé (Nt6) (SEQ ID n° : 27) est fournie ci-après, les sites de restriction étant indiqués entre parenthèses :

La partie de la séquence (Nt6) codant pour le peptide signal d'une chitinase de haricot (SEQ ID n° : 29) ainsi qu'en vis-à-vis la séquence d'acides aminés de ce peptide signal (SEQ ID n° : 28), sont précisées ci-après :

### b) Préparation de la séquence promotrice comprenant le promoteur 35S du virus de la mosaïque du chou-fleur

A partir du plasmide pBI121 (Clontech) par coupure à l'aide des endonucléases HindIII et BamHI, puis électrophorèse sur gel d'agarose, le fragment HindIII-BamHI d'environ 900 pb contenant le promoteur 35S est isolé. Ce fragment est recoupé par HindII. Le fragment d'environ 410 pb, portant le site BamHI, est traité par l'ADN ligase T4 en présence d'un linker HindIII (séquence synthétique contenant un site HindIII). Après coupure par l'endonucléase HindIII et électrophorèse sur gel d'agarose, le fragment HindIII-BamHI résultant (d'environ 420 pb) est isolé et purifié.

### c) Préparation de la séquence terminatrice comprenant le terminateur de la nopaline synthase (NOS) d'Agrobacterium tumefaciens

A partir du plasmide pBI121 (Clontech) par coupure à l'aide des enzymes de restriction SacI et EcoRI, puis électrophorèse sur gel d'agarose, un fragment de 250 pb environ, renfermant le terminateur de la nopaline synthase, a été isolé.

### Clonage dans le vecteur binaire pBIN19

On a ligué à l'aide de l'ADN ligase T4 la séquence promotrice, (cf. section 12 b)), la séquence codante de l'ADN complémentaire de la chitinase des constructions 1 et 2 et la séquence terminatrice (cf. section 12 c)) dans le vecteur binaire pBIN19 ouvert à l'aide des endonucléases HindIII et EcoRI. Ce vecteur porte deux gènes de résistance à la kanamycine, l'un pouvant s'exprimer dans les bactéries, l'autre situé immédiatement en amont du gène recombinant complet (cf. Bevan, 1984, Nucl. Ac. Res., 12, 8711-8721) pouvant être transféré aux cellules végétales. Le gène de résistance à la kanamycine servira de marqueur de sélection au cours des étapes de transformation et d'analyse de la descendance des plantes transformées.

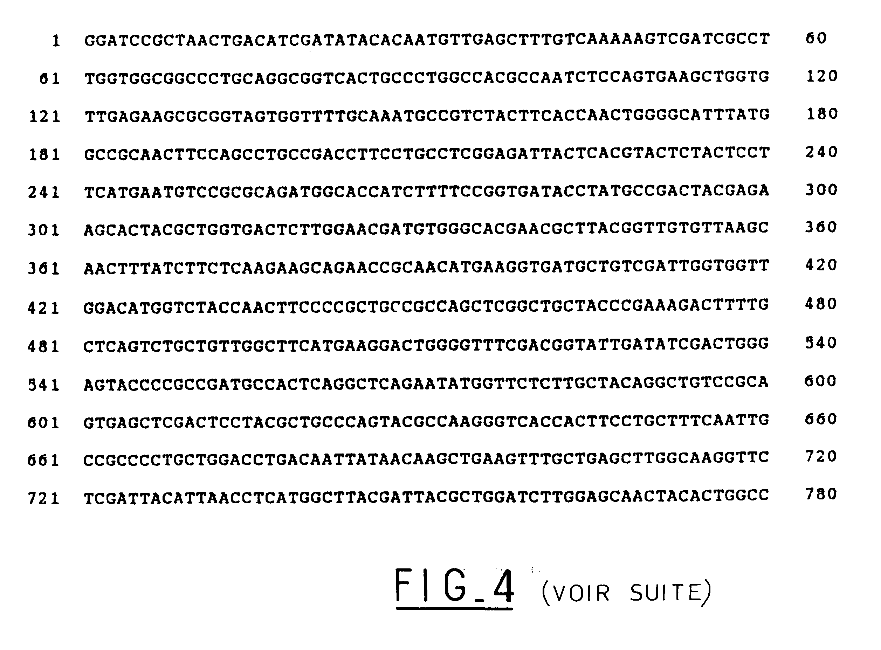

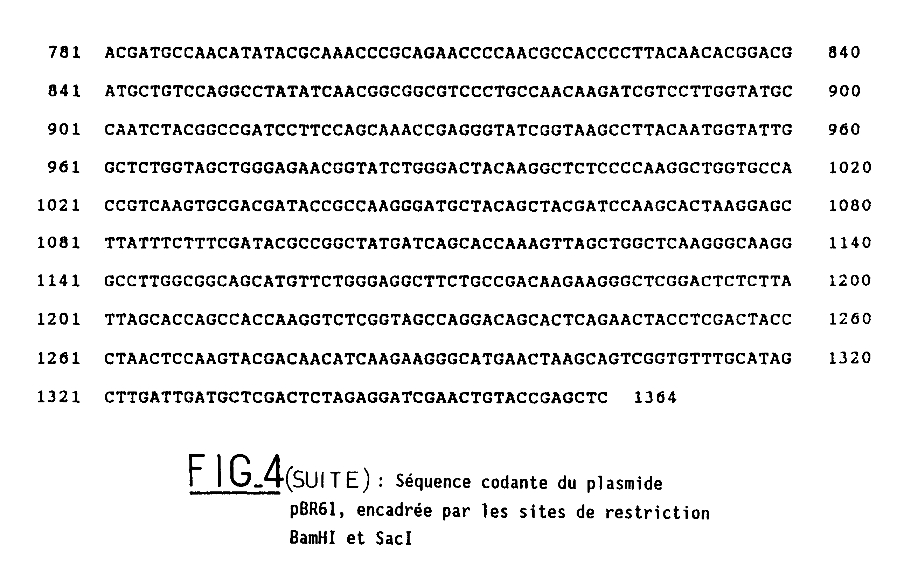

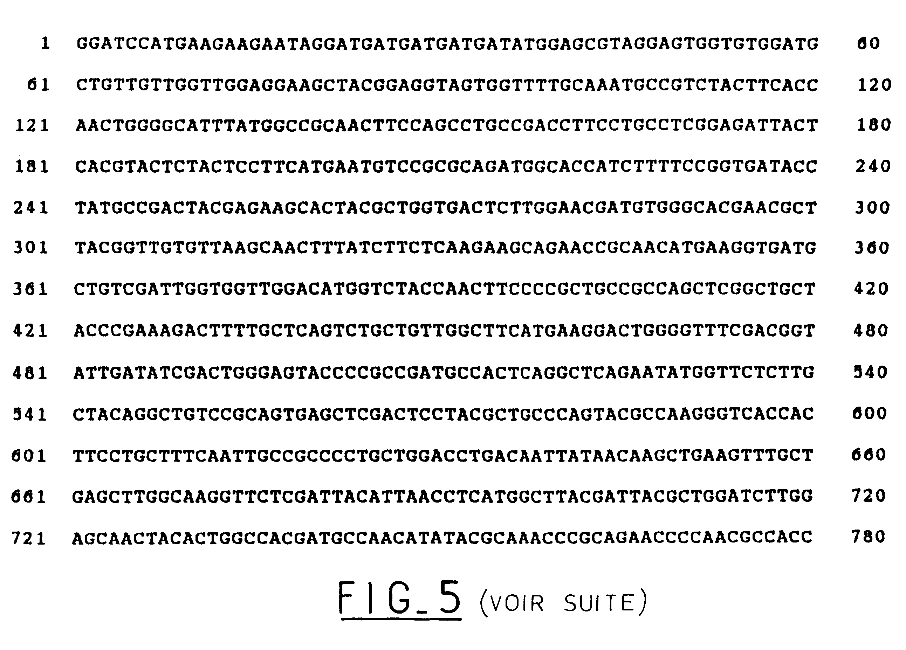

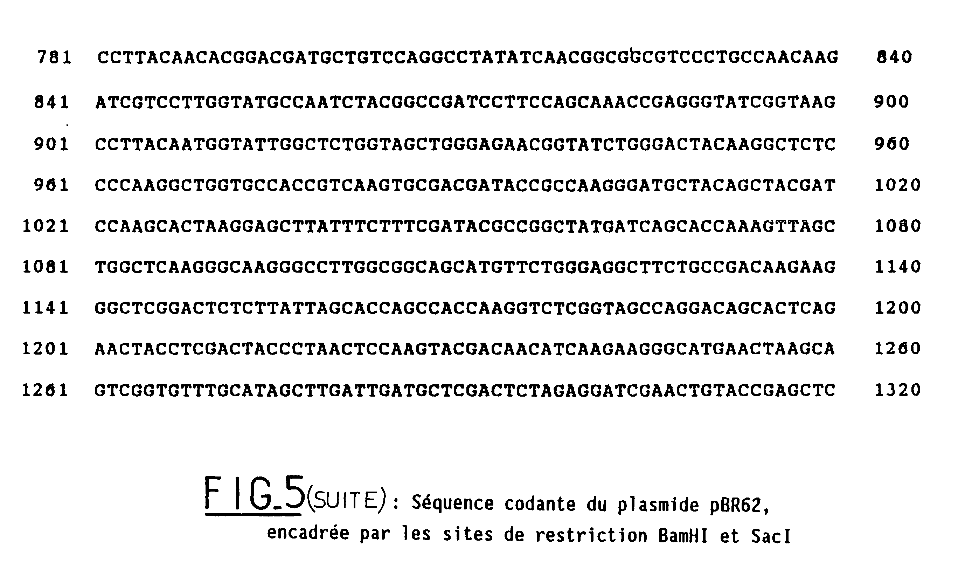

Le vecteur obtenu est appelé plasmide pBR61 lorsqu'il contient la construction 1 et plasmide pBR62 lorsqu'il contient la construction 2. La séquence nucléotidique du gène recombinant complet a été vérifiée par séquençage pour chacun des plasmides pBR61 et pBR62. La séquence codante de ce gène, encadrée par les sites de restriction BamHI et SacI est représentée sur la figure 4 pour le plasmide pBR61 et sur la figure 5 pour le plasmide pBR62. Ces plasmides sont clonés dans la souche E. coli JM109 (Stratagène).

### Section 13 : Transfert dans Agrobacterium tumefaciens des plasmides pBR61 et pBR62 contenant un gène codant pour la chitinase d'Aphanocladium album

### a) Transfert dans Agrobacterium tumefaciens

Ce transfert est réalisé comme décrit ci-après par conjugaison triparentale entre la souche E. coli JM109 (Sambrook et al., op cité) contenant les vecteurs pBR61 ou pBR62 et la souche Agrobacterium tumefaciens LBA4404 (Clontech) à l'aide de la souche E. coli HB101 contenant le plasmide mobilisateur pRK2013 (D.M. Figurski et al., 1979, Proc. Ntl. Acad. Sci. USA, 76, 1648-1652).

La souche E. coli JM109 contenant le plasmide pBR61 ou pBR62 et une souche E. coli HB101 (Clontech) contenant le plasmide mobilisateur pRK2013 sont cultivées à 37°C dans du milieu Luria (Gibco) en présence de 25 mg/l de kanamycine.

La souche Agrobacterium tumefaciens LBA4404 est cultivée à 28°C dans du milieu Luria (Gibco) en présence de 100 mg/l de rifampicine (elle est résistante à cet antibiotique) ; 200 µl de chacune des trois cultures sont mélangés, étalés sur du milieu Luria gélosé (Gibco) et incubés pendant une nuit à 28°C. Les bactéries sont ensuite remises en suspension dans 5 ml de milieu Luria et des parties aliquotes sont étalées sur des boîtes de Pétri contenant un milieu minimum gélosé (décrit dans "Plant Molecular Biology Manual", Gelvin et al., Kluwer Academic Press, 1988) en présence de 100 mg/l de rifampicine et 25 mg/l de kanamycine. Dans ces conditions, seules poussent les colonies d'Agrobacterium tumefaciens ayant intégré le plasmide pBR61 ou pBR62 (les souches d'E. coli ne peuvent pas pousser dans ces conditions). Celles-ci contiennent le gène recombinant de chitinase dans un contexte permettant sa réplication.

La résistance aux deux antibiotiques des colonies sélectionnées est vérifiée en repiquant celles-ci sur le même milieu de sélection deux fois de suite. La présence du gène recombinant de chitinase dans Agrobacterium tumefaciens est vérifiée par la méthode de Southern Blot sur une préparation d'ADN total (Lyse des cellules, purification de l'ADN par extraction à l'aide du mélange phénol/chloroforme, selon le protocole décrit par Gelvin dans l'ouvrage cité ci-dessus, coupure de l'ADN purifié à l'aide d'enzymes de restriction, électrophorèse sur gel d'agarose, transfert sur membrane et hybridation selon les techniques bien connues de l'homme de l'art).

### b) Transfert dans Agrobacterium rhizogenes

Ce transfert est réalisé de la même façon que le transfert dans Agrobacterium tumefaciens décrit en a), avec la souche Agrobacterium rhizogenes A4 décrite par GUERCHE et al., (1987), Mol. gen. genet., 206, 382.

### Section 14 : Obtention de plantes de tabac transformées

Du tabac Nicotiana tabacum cultivé in vitro a été infecté par Agrobacterium tumefaciens contenant le plasmide pBR61 ou pBR62 selon la procédure de Horsch et al., bien connue de l'homme du métier (Horsch R.B. et al., 1985, Science 227, 1229-1231), dont les principales étapes sont exposées ci-après.

Des disques de feuilles de plantes axéniques de tabac N. tabacum (variété Wisconsin Havana 38 sensible aux champignons pathogènes) sont incubés dans une culture d'A. tumefaciens hébergeant le plasmide pBR61 ou pBR62. Les disques égouttés sur papier Whatman ont été transférés sur des milieux de culture en boîtes de Pétri afin de multiplier les cellules transformées de façon à obtenir des cals, puis des plantes régénérées.

### Section 15 : Mise en évidence de l'expression de la chitinase d'A. album dans les cals et dans les feuilles de plantes de tabac transformés

### a) Préparation des extraits bruts de protéines de tabac transformées

Les fragments de tissus (cals ou feuilles de plantes) ont été congelés dans l'azote liquide, réduits en poudre et stockés à -20°C. La poudre a été extraite à 4°C en présence d'un tampon acétate d'ammonium 0,1 M pH 5,2 et soumise à une centrifugation à 10 000 g. La concentration des protéines totales a été déterminée sur les surnageants, appelés ci-après les extraits bruts de protéines, en suivant la technique de Bradford (Bradford, M.M., 1976, Anal. Biochem., 72, 248-254).

### b) Mise en évidence de la chitinase recombinante par immuno-empreinte (Western blot)

On soumet les extraits bruts de protéines de différents cals (ou feuilles de plantes) transformés et des cals (ou feuilles de plantes) non transformés (témoins) à un Western blot, technique bien connue de l'homme de l'art et décrite notamment par H. Towbin et al., Proc. Ntl. Acad. Sci. USA, 76, 1979, 4350-4354, qui comprend les étapes suivantes :
- dénaturation par chauffage à 100° pendant 10 min dans un tampon, dénommé tampon de charge constitué de Tris HCl 0,125 M pH 6,8, SDS 4 %, bleu de bromophénol 0,002 %, glycérol 20 %, β-mercaptoéthanol 10 % (selon le protocole décrit par Laemmli, U.K. Laemmli, Nature, 227, 1970, 680-685) ;
- séparation électrophorétique des différentes protéines contenues dans le solubilisat selon le protocole décrit par Laemmli (réf. ci-dessus) ;
- électrotransfert desdites protéines contenues dans le gel sur une membrane en PVDF (selon la technique de H. Towbin et al., Proc. Natl. Acad. Sci. USA, 76, 1979, 4350-4354).

L'immunodétection est réalisée selon un protocole qui comprend les étapes suivantes :
- saturation de la membrane PVDF sur laquelle les protéines ont été transférées par incubation pendant au minimum 2 h à 37°C dans une solution de gélatine à 3 %,
- 3 lavages dans du tampon phosphate salin contenant 0,05 % de détergent Tween 20,
- incubation (pendant 1 h à 37°C) en présence de l'immunsérum préparé précédemment (contenant les anticorps polyclonaux reconnaissant la protéine recombinante) dilué au 1/10 000 dans du tampon phosphate salin,
- 3 lavages dans du tampon phosphate salin contenant 0,05 % de détergent Tween 20.

Le complexe antigène-anticorps est ensuite révélé à l'aide d'un système streptavidine-biotine conjugué à la phosphatase alcaline avec le kit RPN 23 d'Amersham ("Blotting détection kit"), utilisé selon les indications du fabricant.

L'empreinte obtenue montre, pour les cals et pour les feuilles de plantes de tabac transformés par chacun des deux plasmides pBR61 et pBR62, la présence d'une protéine de poids moléculaire apparent d'environ 41 ± 3 kDa, absente des cals et des feuilles de plantes de tabac témoins. Cette protéine a le même poids moléculaire apparent que la chitinase d'A. album purifiée, obtenue dans la section 1.

### c) Mise en évidence de l'activité chitinolytique de la chitinase recombinante

L'activité chitinolytique des 5 extraits bruts de protéines de cals et de feuilles de plantes de tabac transformés par chacun des deux plasmides pBR61 et pBR62 et des 5 extraits bruts de protéines de cals et de feuilles de plantesde tabac non transformés est mesurée selon laméthode radiochimique de Molano et al., décrite section 1 b1. Compte tenu de l'activité chitinolytique endogène, un moyen simple de montrer l'activité chitinolytique de la chitinase recombinante est d'inactiver spécifiquement celle-ci par des anticorps et deconstater la baisse d'activité chitinolytique totale des extraits.

10 µl de ces extraits bruts sont mis en présence, pendant 5 min à température ambiante, de 1 µl des anticorps polyclonaux dirigés contre la chitinase d'A. album (préparés dans la section 1 c)). L'activité chitinolytique de ces mélanges extraits bruts/anticorps est également mesurée par la méthode radiochimique de Molano et al. (section 1 b)).

L'activité chitinolytique des extraits bruts de protéines de cals et de feuilles de plantes de tabac transformés par chacun des plasmides pBR61 et pBR62 est significativement plus élevée que celle des extraits de cals et de feuilles de plantes de tabac témoins. Après incubation en présence d'anticorps contre la chitinase d'A. album, l'activité des extraits de cals et de feuilles de plantes de tabac transformés par chacun des plasmides pBR61 et pBR62 diminue, alors que celle des extraits de cals et de feuilles de plantes de tabac témoins n'est pas affectée.

La chitinase d'A. album recombinante exprimée dans le tabac possède donc une activité chitinolytique.

### d) Purification de la chitinase recombinante et détermination de sa séquence aminoterminale :

### d1) Purification de la chitinase recombinante :

La protéine recombinante a été purifiée à partir des extraits bruts de protéines de feuilles de plantes de tabac transformées par chacun des plasmides pBR61 et pBR62, par précipitation au sulfate d'ammonium et chromatographie en phase liquide selon la technique FPLC de Pharmacia sur une colonne échangeuse de cations à base de polymère synthétique sur agarose réticulé, selon le protocole décrit ci-après :

### Protocole de purification de la chitinase recombinante

### Etape 1 :

L'extrait protéique est précipité par le sulfate d'ammonium (60 % de saturation). Les protéines ayant précipité sont récupérées par centrifugation (15 000 g pendant 30 min), solubilisées dans une solution tampon (acétate d'ammonium 100 mM pH 5,2) et dialysées une nuit à 4°C contre une solution tampon acétate d'ammonium 100 mM pH 5,2.

Extemporanément, la concentration de la solution tampon de l'extrait protéique est ramenée à 10 mM par passage sur des mini-colonnes prêtes à l'emploi (PD10. Pharmacia).

### Etape 2 :

L'extrait protéique est ensuite purifié par chromatographie sur une colonne échangeuse d'ions à base de polymère synthétique (colonne Mono-S Pharmacia) à l'aide d'une technique FPLC (Pharmacia).

L'extrait est déposé sur la colonne Mono-S équilibrée avec un tampon acétate d'ammonium 10 mM pH 5,2. Les protéines retenues sur la colonne sont éluées par un gradient linéaire de 10 à 500 mM d'acétate d'ammonium.

A chaque étape, la chitinase recombinante est identifiée par son poids moléculaire (Electrophorèse sur gel de polyacrylamide en présence de SDS-révélation à l'argent), par son immuno-empreinte (cf. paragraphe b) de la section 8) et son activité, mesurée par la méthode radiochimique décrite dans le paragraphe b1 de la section 1.

### d2) Essai de détermination de la séquence aminoterminale de la chitinase recombinante

Après purification de la chitinase recombinante selon le protocole décrit ci-dessus, le séquençage de l'extrémité aminoterminale a été réalisé. Les échantillons à traiter sont portés à la surface d'un filtre PVDF (polyvinylidenedifluoride) par électrotransfert selon la méthode décrite par H. Towbin et al., Proc. Ntl. Acad. Sci. USA (1979), 4350-4354, après électrophorèse sur gel de polyacrylamide en présence de SDS. Le filtre est introduit dans un séquenceur de protéines (modèle 470 A commercialisé par la société Applied Biosystems (E.U.A.)) équipé d'un chromatographe (modèle 430 d'Applied Biosystems) qui analyse en continu les dérivés phénylthiohydantoïques formés après chaque cycle de dégradation.

Pour la protéine recombinante obtenue à partir de feuilles de tabac transformées par chacun des deux plasmides pBR61 et pBR62 on obtient la séquence aminoterminale suivante (acides aminés 1-12 de SEQ ID n° : 1) :

Le clivage du peptide signal se produit donc au site attendu.

### Section 16 : Obtention de plantes de colza transformées

La transformation est réalisée selon le protocole de P. Guerche et al. (P. Guerche et al., 1987, Mol. Gen. Genet., 206, 382). Les différents milieux de culture sont ceux décrits par Pelletier et al. (Pelletier et al., 1983, Mol. Gen. Genet., 191-244). Leur composition sera explicitée par la suite (tableau VI).

### a) Obtention de racines transformées

Des segments de tige sont prélevés sur l'extrémité apicale de plantes de colza (Brassica napus : variétés de printemps Brutor, Westar et variétés d'hiver) de 1 m de haut environ. Ces segments sont stérilisés en surface, rincés dans de l'eau stérile, découpés en segments de 1,5 cm de long environ et placés dans un tube contenant le milieu A.

L'inoculation de l'extrémité de ce segment est effectuée par dépôt d'une suspension de la souche d'Agrobacterium rhizogenes contenant le plasmide pBR61 ou pBR62.

Des racines transformées apparaissent sur le segment de tige au bout de 1 à 2 semaines, elles sont prélevées et placées sur le milieu B gélosé (15 g/l) et complémenté par 500 µg de céfotaxime/ml.

### b) Régénération de plantes transformées

Des fragments de racines sont incubés pendant 15 jours sur le milieu D contenant 3 mg/l d'acide 2,4-dichlorophénoxyacétique, puis placés sur le milieu RCC d'induction de bourgeons. Des plantes racinées sont ensuite obtenues par passage des bourgeons sur les milieux F et G (tableau VI ci-après).

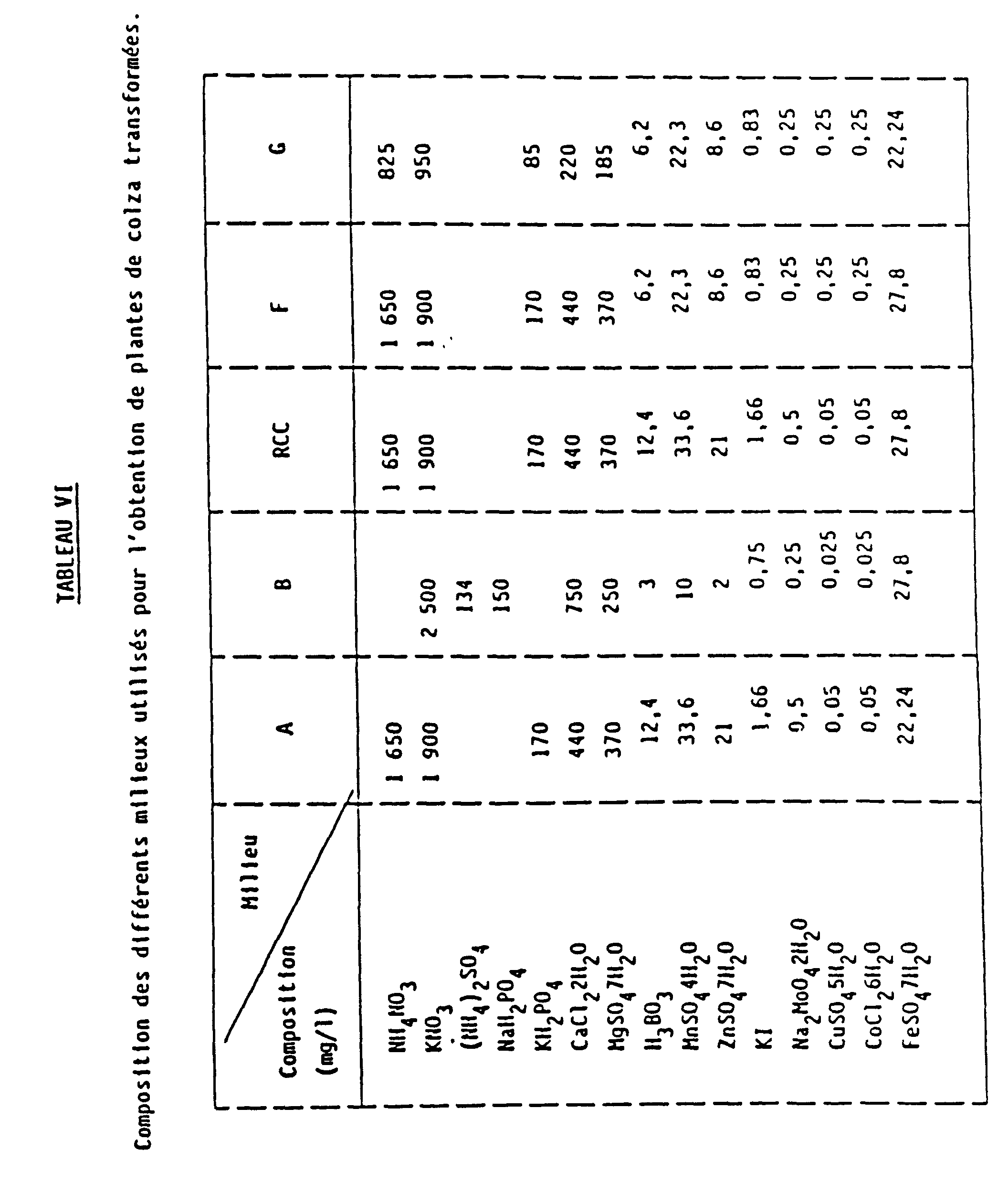

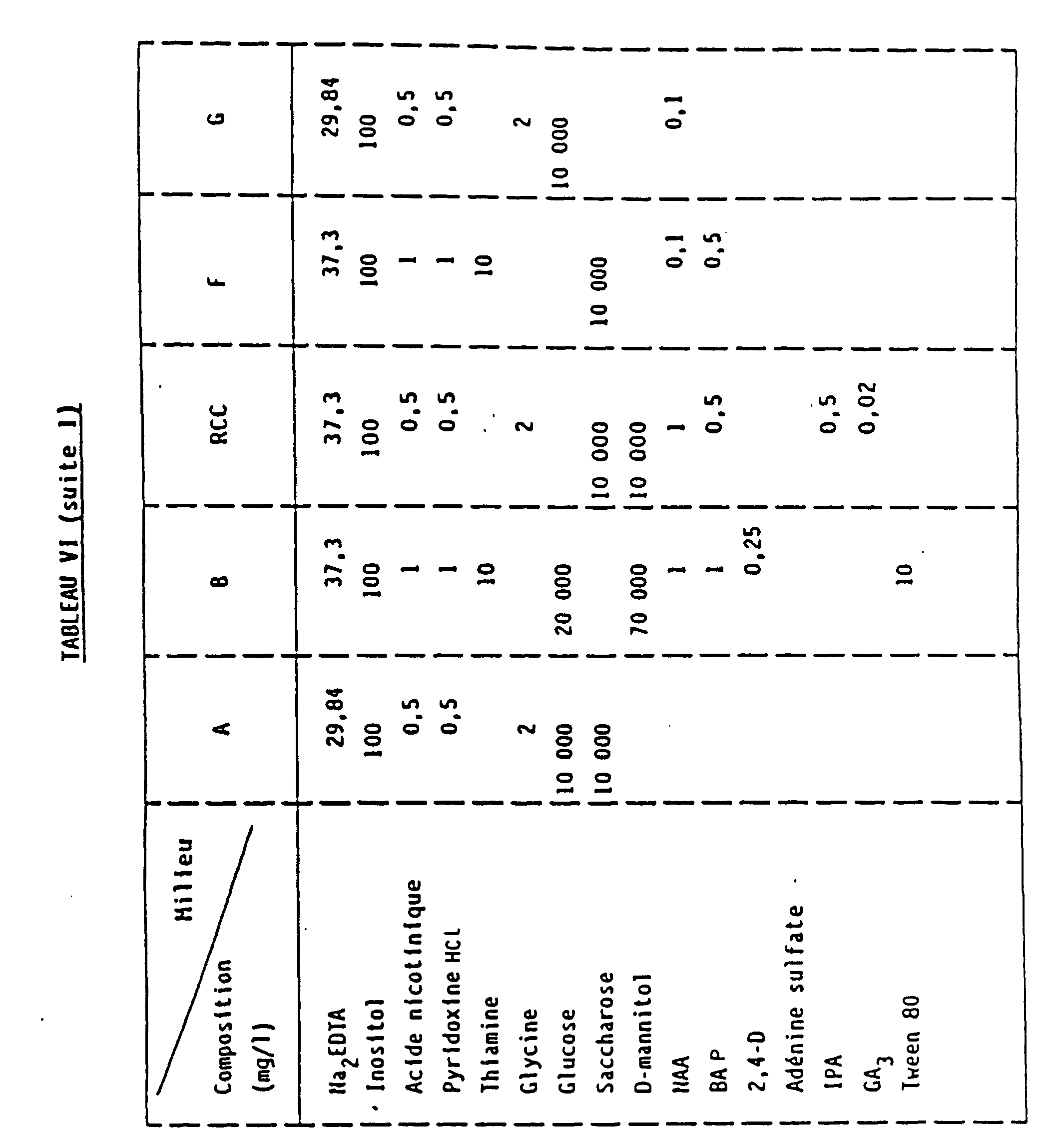

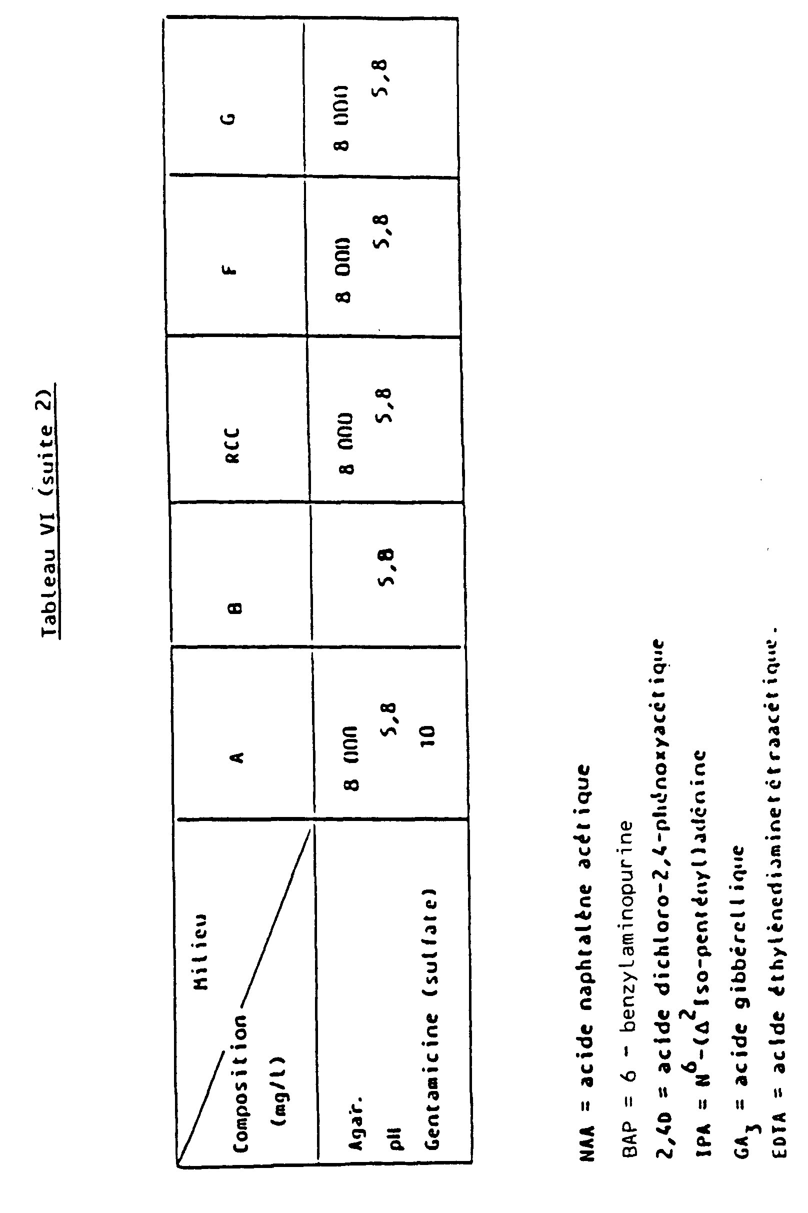

### Section 17 : Mise en évidence de l'expression de la chitinase d'A. album dans des racines transformées de colza

### a) Préparation des extraits bruts de protéines de racines transformées de colza

Les extraits sont préparés comme indiqué dans la section 15 a).

### b) Mise en évidence de la chitinase recombinante par immuno-empreinte (Western blot)

Le protocole suivi est celui décrit précédemment dans la section 15 b).

L'empreinte obtenue montre la présence d'une protéine de poids moléculaire apparent d'environ 41 ± 3 kDa présente dans les racines transformées par le plasmide pBR61 ou le plasmide pBR62, absente des extraits de racines non transformées servant de témoin. Cette protéine a le même poids moléculaire apparent que la chitinase naturelle d'A. Album purifiée, obtenue dans la section 1.

## Revendications

1. ADN recombinant, caractérisé en ce qu'il code pour une protéine à activité endochitinase ou pour un précurseur de cette dernière, qui comprend la séquence (a1) suivante (SEQ ID N° : 1) :

2. ADN recombinant selon la revendication 1, caractérisé en ce qu'il comporte, en amont de la séquence (a1), une séquence signal.

3. ADN recombinant selon la revendication 2, caractérisé en ce que la séquence signal est une séquence codant pour le peptide signal de séquence (a2) suivante (SEQ ID N° : 4) :

4. ADN recombinant selon la revendication 2 ou 3, caractérisé en ce que le peptide signal codé par la séquence signal est séparé de la séquence (a1) de la protéine codée, par le peptide de séquence (a3) suivante (SEQ ID N° : 5) :

5. ADN recombinant selon la revendication 2, caractérisé en ce que la séquence signal est une séquence codant pour le peptide signal de séquence (a5) suivante (SEQ ID N° : 28):

6. ADN recombinant selon l'une des revendications 1 à 5, caractérisé en ce qu'il comprend une séquence promotrice comportant le promoteur 35S du virus de la mosaïque de chou-fleur.

7. ADN recombinant selon l'une des revendications 1 à 6, caractérisé en ce qu'il comprend une séquence terminatrice comportant le terminateur de la nopaline synthase d'Agrobacterium tumefaciens.

8. ADN recombinant selon l'une des revendications 1 à 7, caractérisé en ce que la séquence nucléotidique codant pour la séquence d'acides aminés (a1) est la séquence (Nal) suivante (SEQ ID N° : 6) :

9. ADN recombinant selon la revendication 3, caractérisé en ce que la séquence nucléotidique codant pour la séquence d'acides aminés (a2) est la séquence (Na2) suivante (SEQ ID N° : 7) :

10. ADN recombinant selon la revendication 4, caractérisé en ce que la séquence nucléotidique codant pour la séquence d'acides aminés (a3) est la séquence (Na3) suivante (SEQ ID N° : 8) :

11. ADN recombinant selon la revendication 5, caractérisé en ce que la séquence nucléotidique codant pour la séquence d'acides aminés (a5) est la séquence (Na5) suivante (SEQ ID N° : 29) :

12. Bactérie, caractérisée en ce qu'elle contient, avec les moyens nécessaires à sa réplication et son expression, l'ADN recombinant selon l'une des revendications 1 à 4.

13. Levure, caractérisée en ce qu'elle contient, avec les moyens nécessaires à sa réplication et son expression, l'ADN recombinant selon l'une des revendications 1 à 4.

14. Champignon filamenteux, caractérisé en ce qu'il contient, avec les moyens nécessaires à sa réplication et son expression, l'ADN recombinant selon l'une des revendications 1 à 4.

15. Cellule végétale, caractérisée en ce qu'elle est transformée par un ADN recombinant selon l'une des revendications 1 à 11, avec les moyens nécessaires à son expression.

16. Cellule végétale selon la revendication 15, caractérisée en ce qu'elle appartient à l'une des espèces Nicotiana tabacum, Helianthus annuus et Brassica napus.

17. Plante ou partie de plante, caractérisée en ce qu'elle contient un ADN recombinant selon l'une des revendications 1 à 11, avec les moyens nécessaires à son expression.

18. Plante ou partie de plante selon la revendication 17, caractérisée en ce qu'elle appartient à l'une des espèces Nicotiana tabacum, Helianthus annuus et Brassica napus.

19. Partie de plante selon la revendication 17 ou 18, caractérisée en ce qu'elle est apte à former une nouvelle plante complète ou à produire des semences.

20. Partie de plante selon la revendication 19, caractérisée en ce qu'elle est une semence.

21. Protéine à activité endochitinase, caractérisée en ce qu'elle est susceptible d'être obtenue à l'aide d'un ADN recombinant selon l'une des revendications 1 à 11.

22. Protéine selon la revendication 21, caractérisée en ce qu'elle comprend la séquence (a1).

23. Protéine selon la revendication 21 ou 22, caractérisée en ce qu'elle a un poids moléculaire apparent de 41 ± 3 kDa, déterminé par électrophorèse sur gel de polyacrylamide en présence de SDS.

24. Protéine selon l'une des revendications 21 à 23, caractérisée en ce qu'elle est N-glycosylée.

25. Protéine selon la revendication 21 ou 22, caractérisée en ce qu'elle a un poids moléculaire apparent de 39 ± 3 kDa, déterminé par électrophorèse sur gel de polyacrylamide en présence de SDS.

26. Procédé pour préparer la protéine selon la revendication 21 ou 22, caractérisé en ce qu'il comprend la mise en culture de cellules végétales, de cals végétaux, de plantes, ou de parties de plantes contenant, avec les moyens nécessaires à son expression, un ADN recombinant selon l'une des revendications 1 à 11, la lyse de ceux-ci, l'isolement et la purification de cette protéine.

## Claims

1. A recombinant DNA characterized in that it codes for a protein with endochitinase activity or for a precursor of said protein, said protein comprising the following sequence (a1) (SEQ ID N°:1) :

2. The recombinant DNA according to claim 1, characterized in that it contains a signal sequence upstream of the sequence (a1).

3. The recombinant DNA according to claim 2, characterized in that the signal sequence is a sequence coding for the signal peptide of the following sequence (a2) (SEQ ID N°:4) :

4. The recombinant DNA according to claim 2 or 3, characterized in that the signal peptide coded by the signal sequence is separated from the sequence (a1) of the coded protein by the peptide of the following sequence (a3) (SEQ ID N°:5) :

5. The recombinant DNA according to claim 2, characterized in that the signal sequence is a sequence coding for the signal peptide of the following sequence (a5) (SEQ ID N°:28) :

6. The recombinant DNA according to one of claims 1 to 5, characterized in that it comprises a promoter sequence containing the 35S promoter of cauliflower mosaic virus.

7. The recombinant DNA according to one of claims 1 to 6, characterized in that it comprises a terminator sequence containing the terminator of the nopaline synthase of *Agrobacterium tumefaciens.*

8. The recombinant DNA according to one of claims 1 to 7, characterized in that the nucleotide sequence coding for the amino acid sequence (a1) is the following sequence (Na1) (SEQ ID N°:6) :

9. The recombinant DNA according to claim 3, characterized in that the nucleotide sequence coding for the amino acid sequence (a2) is the following sequence (Na2) (SEQ ID N°:7) :

10. The recombinant DNA according to claim 4, characterized in that the nucleotide sequence coding for the amino acid sequence (a3) is the following sequence (Na3) (SEQ ID N°:8) :

11. The recombinant DNA according to claim 5, characterized in that the nucleotide sequence coding for the amino acid sequence (a5) is the following sequence (Na5) (SEQ ID N°:29) :

12. A bacterium, characterized in that it contains the recombinant DNA according to one of claims 1 to 4, together with the means necessary for its replication and its expression.

13. A yeast, characterized in that it contains the recombinant DNA according to one of claims 1 to 4, together with the means necessary for its replication and its expression.

14. A filamentous fungus, characterized in that it contains the recombinant DNA according to one of claims 1 to 4, together with the means necessary for its replication and its expression.

15. A plant cell, characterized in that it is transformed by a recombinant DNA according to one of claims 1 to 11, together with the means necessary for its expression.

16. The plant cell according to claim 15, characterized in that it belongs to one of the species *Nicotiana tabacum*, *Helianthus annuus or Brassica napus.*

17. A plant or a plant part, characterized in that it contains a recombinant DNA according to one of claims 1 to 11, together with the means necessary for its expression.

18. The plant or plant part according to claim 17, characterized in that it belongs to one of the species *Nicotiana tabacum*, *Helianthus annuus* or *Brassica napus.*

19. The plant part according to claim 17 or claim 18, characterized in that it is capable of forming a complete new plant or of producing seeds.

20. The plant part according to claim 19, characterized in that it is a seed.

21. A protein with endochitinase activity, characterized in that it can be obtained with the aid of a recombinant DNA according to one of claims 1 to 11.

22. The protein according to claim 21, characterized in that it comprises the sequence (a1).

23. The protein according to claim 21 or claim 22, characterized in that it has an apparent molecular weight of 41 ± 3 kDa, as determined by electrophoresis on a polyacrylamide gel in the presence of SDS.

24. The protein according to one of claims 21 to 23, characterized in that it is N-glycosylated.

25. The protein according to claim 21 or claim 22, characterized in that it has an apparent molecular weight of 39 ± 3 kDa, as determined by electrophoresis on a polyacrylamide gel in the presence of SDS.

26. A process for the preparation of the protein according to claim 21 or claim 22, characterized in that it comprises culturing plant cells, plant calluses, plants or plant parts containing a recombinant DNA according to one of claims 1 to 11, together with the means necessary for its expression, lysing them and isolating and purifying this protein.

## Patentansprüche

1. Rekombinante DNA, dadurch gekennzeichnet, dass sie für ein Protein mit Endochitinase-Aktivität oder einen Vorläufer desselben codiert, das bzw. der die folgende Sequenz (a1) (SEQ ID No.: 1) aufweist:

2. Rekombinante DNA nach Anspruch 1, dadurch gekennzeichnet, dass sie stromaufwärts der Sequenz (a1) eine Signalsequenz aufweist.

3. Rekombinante DNA nach Anspruch 2, dadurch gekennzeichnet, dass die Signalsequenz eine Sequenz ist, die für das Signalpeptid mit der folgenden Sequenz (a2) (SEQ ID No.: 4) codiert:

4. Rekombinante DNA nach Anspruch 2 oder 3, dadurch gekennzeichnet, dass das von der Signalsequenz codierte Signalpeptid von der Sequenz (a1) des codierten Proteins getrennt ist, und zwar durch das Peptid mit der folgenden Sequenz (a3) (SEQ ID No.: 5):

5. Rekombinante DNA nach Anspruch 2, dadurch gekennzeichnet, dass die Signalsequenz eine Sequenz ist, die für das Signalpeptid mit der folgenden Sequenz (a5) (SEQ ID No.: 28) codiert:

6. Rekombinante DNA nach einem der Ansprüche 1 bis 5, dadurch gekennzeichnet, dass sie eine Promotorsequenz aufweist, die den Promotor 35S des Blumenkohl-Mosaikvirus umfasst.

7. Rekombinante DNA nach einem der Ansprüche 1 bis 6, dadurch gekennzeichnet, dass sie eine Terminationssequenz aufweist, die den Terminator der Nopalinsynthase von Agrobacterium tumefaciens umfasst.

8. Rekombinante DNA nach einem der Ansprüche 1 bis 7, dadurch gekennzeichnet, dass die Nucleotidsequenz, die für die Aminosäuresequenz (a1) codiert, die folgende Sequenz (Na1) (SEQ ID No.: 6) ist:

9. Rekombinante DNA nach Anspruch 3, dadurch gekennzeichnet, dass die Nucleotidsequenz, die für die Aminosäuresequenz (a2) codiert, die folgende Sequenz (Na2) (SEQ ID No.: 7) ist:

10. Rekombinante DNA nach Anspruch 4, dadurch gekennzeichnet, dass die Nucleotidsequenz, die für die Aminosäuresequenz (a3) codiert, die folgende Sequenz (Na3) (SEQ ID No.: 8) ist:

11. Rekombinante DNA nach Anspruch 5, dadurch gekennzeichnet, dass die Nucleotidsequenz, die für die Aminosäuresequenz (a5) codiert, die folgende Sequenz (Na5) (SEQ ID No.: 29) ist:

12. Bakterium, dadurch gekennzeichnet, dass es die rekombinante DNA nach einem der Ansprüche 1 bis 4 zusammen mit den für ihre Replikation und Expression notwendigen Mitteln enthält.

13. Hefe, dadurch gekennzeichnet, dass sie die rekombinante DNA nach einem der Ansprüche 1 bis 4 zusammen mit den für ihre Replikation und Expression notwendigen Mitteln enthält.

14. Fadenpilz, dadurch gekennzeichnet, dass er die rekombinante DNA nach einem der Ansprüche 1 bis 4 zusammen mit den für ihre Replikation und Expression notwendigen Mitteln enthält.

15. Pflanzliche Zelle, dadurch gekennzeichnet, dass sie durch eine rekombinante DNA nach einem der Ansprüche 1 bis 11 zusammen mit den für ihre Expression notwendigen Mitteln transformiert ist.

16. Pflanzliche Zelle nach Anspruch 15, dadurch gekennzeichnet, dass sie zu einer der Arten Nicotiana tabacum. Helianthus annuus und Brassica napus gehört.

17. Pflanze oder Pflanzenteil, dadurch gekennzeichnet, dass sie bzw. er eine rekombinante DNA nach einem der Ansprüche 1 bis 11 zusammen mit den für ihre Expression notwendigen Mitteln enthält.

18. Pflanze oder Pflanzenteil nach Anspruch 17, dadurch gekennzeichnet, dass sie bzw. er zu einer der Arten Nicotiana tabacum, Helianthus annuus und Brassica napus gehört.

19. Pflanzenteil nach Anspruch 17 oder 18, dadurch gekennzeichnet, dass er zur Bildung einer neuen vollständigen Pflanze oder zur Produktion von Samen geeignet ist.

20. Pflanzenteil nach Anspruch 19, dadurch gekennzeichnet, dass er ein Samen ist.

21. Protein mit Endochitinase-Aktivität, dadurch gekennzeichnet, dass es mit Hilfe einer rekombinanten DNA nach einem der Ansprüche 1 bis 11 erhalten werden kann.

22. Protein nach Anspruch 21, dadurch gekennzeichnet, dass es die Sequenz (a1) aufweist.

23. Protein nach Anspruch 21 oder 22, dadurch gekennzeichnet, dass es ein scheinbares Molekulargewicht von 41 ± 3 kDA aufweist, bestimmt mittels Elektrophorese auf Polyacrylamidgel in Gegenwart von SDS.

24. Protein nach einem der Ansprüche 21 bis 23, dadurch gekennzeichnet, dass es N-glycosyliert ist.

25. Protein nach Anspruch 21 oder 22, dadurch gekennzeichnet, dass es ein scheinbares Molekulargewicht von 39 ± 3 kDA aufweist, bestimmt mittels Elektrophorese auf Polyacrylamidgel in Gegenwart von SDS.

26. Verfahren zur Herstellung des Proteins nach Anspruch 21 oder 22, dadurch gekennzeichnet, dass es das Kultivieren von pflanzlichen Zellen, pflanzlichem Kallus, Pflanzen oder Pflanzenteilen, die eine rekombinante DNA nach einem der Ansprüche 1 bis 11 zusammen mit den für ihre Expression notwendigen Mitteln enthalten, das Lysieren derselben, das Isolieren und das Reinigen dieses Proteins umfasst.


### SEQUENCE LISTING

(1) GENERAL INFORMATION:
   (i) APPLICANT: ELF SANOFI
   (ii) TITLE OF INVENTION: Recombinant DNA coding for a protein having endochitinase activity
   (iii) NUMBER OF SEQUENCES: 29
   (iv) CORRESPONDENCE ADDRESS:
      (A) ADDRESSEE:Cabinet Beau de Lomenie
      (B) STREET: 55, Rue d'Amsterdam
      (C) CITY: PARIS
      (E) COUNTRY: FRANCE
      (F) ZIP: 75008
   (v) COMPUTE READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vii) PRIOR APPLICATION DATA:
      (A) APPLICATION NUMBER: FR 91 11072
      (B) FILING DATE: 06-SEP-1991
   (viii) ATTORNEY/AGENT INFORMATION:
      (A) NAME: Marie-Louise GILLARD
      (C) REFERENCE/DOCKET NUMBER: MLG.CSB/H2233-567
   (ix) TELECOMMUNICATION INFORMATION:
      (A) TELEPHONE: 42 80 64 68
      (B) TELEFAX: 48 74 37 60
      (C) TELEX: 650476F
(2) INFORMATION FOR SEQ ID NO:1:
   (i) SEQUENCE CHARACTERISTICS:
      (A) LENGTH: 389 amino acids
      (B) TYPE: amino acid
      (D) TOPOLOGY: linear
   (ii) MOLECULE TYPE: protein
   (vii) IMMEDIATE SOURCE:
      (B) CLONE: protein having endochitinase activity
   (xi) SEQUENCE DESCRIPTION: SEQ ID NO:1:
   Gly Ser Gly Phe Ala Asn Ala Val Tyr Phe Thr Asn Trp Gly Ile Tyr
   1 5 10 15
   Gly Arg Asn Phe Gln Pro Ala Asp Leu Pro Ala Ser Glu Ile Thr His
   20 25 30
   Val Leu Tyr Ser Phe Met Asn Val Arg Ala Asp Gly Thr Ile Phe Ser
   35 40 45
   Gly Asp Thr Tyr Ala Asp Tyr Glu Lys His Tyr Ala Gly Asp Ser Trp
   50 55 60
   Asn Asp Val Gly Thr Asn Ala Tyr Gly Cys Val Lys Gln Leu Tyr Leu
   65 70 75 80
   Leu Lys Lys Gln Asn Arg Asn Met Lys Val Met Leu Ser Ile Gly Gly
   85 90 95
   Trp Thr Trp Ser Thr Asn Phe Pro Ala Ala Ala Ser Ser Ala Ala Thr
   100 105 110
   Arg Lys Thr Phe Ala Gln Ser Ala Val Gly Phe Met Lys Asp Trp Gly
   115 120 125
   Phe Asp Gly Ile Asp Ile Asp Trp Glu Tyr Pro Ala Asp Ala Thr Gln
   130 135 140
   Ala Gln Asn Met Val Leu Leu Leu Gln Ala Val Arg Ser Glu Leu Asp
   145 150 155 160
   Ser Tyr Ala Ala Gln Tyr Ala Lys Gly His His Phe Leu Leu Ser Ile
   165 170 175
   Ala Ala Pro Ala Gly Pro Asp Asn Tyr Asn Lys Leu Lys Phe Ala Glu
   180 185 190
   Leu Gly Lys Val Leu Asp Tyr Ile Asn Leu Met Ala Tyr Asp Tyr Ala
   195 200 205
   Gly Ser Trp Ser Asn Tyr Thr Gly His Asp Ala Asn Ile Tyr Ala Asn
   210 215 220
   Pro Gln Asn Pro Asn Ala Thr Pro Tyr Asn Thr Asp Asp Ala Val Gln
   225 230 235 240
   Ala Tyr Ile Asn Gly Gly Val Pro Ala Asn Lys Ile Val Leu Gly Met
   245 250 255
   Pro Ile Tyr Gly Arg Ser Phe Gln Gln Thr Glu Gly Ile Gly Lys Pro
   260 265 270
   Tyr Asn Gly Ile Gly Ser Gly Ser Trp Glu Asn Gly Ile Trp Asp Tyr
           275                 280                 285
   Lys Ala Leu Pro Lys Ala Gly Ala Thr Val Lys Cys Asp Asp Thr Ala
       290                 295                 300
   Lys Gly Cys Tyr Ser Tyr Asp Pro Ser Thr Lys Glu Leu Ile Ser Phe
   305                 310                 315                 320
   Asp Thr Pro Ala Met Ile Ser Thr Lys Val Ser Trp Leu Lys Gly Lys
                   325                 330                 335
   Gly Leu Gly Gly Ser Met Phe Trp Glu Ala Ser Ala Asp Lys Lys Gly
               340                 345                 350
   Ser Asp Ser Leu Ile Ser Thr Ser His Gln Gly Leu Gly Ser Gln Asp
           355                 360                 365
   Ser Thr Gln Asn Tyr Leu Asp Tyr Pro Asn Ser Lys Tyr Asp Asn Ile
       370                 375                 380
   Lys Lys Gly Met Asn
   385
(2) INFORMATION FOR SEQ ID NO:2:
   (i) SEQUENCE CHARACTERISTICS:
      (A) LENGTH: 89 amino acids
      (B) TYPE: amino acid
      (D) TOPOLOGY: linear
   (ii) MOLECULE TYPE: peptide
   (vii) IMMEDIATE SOURCE:
      (B) CLONE: signal peptide of preproendothiapepsin
   (xi) SEQUENCE DESCRIPTION: SEQ ID NO:2:
   Met Ser Ser Pro Leu Lys Asn Ala Leu Val Thr Ala Met Leu Ala Gly
   1               5                   10                  15
   Gly Ala Leu Ser Ser Pro Thr Lys Gln His Val Gly Ile Pro Val Asn
               20                  25                  30
   Ala Ser Pro Glu Val Gly Pro Gly Lys Tyr Ser Phe Lys Gln Val Arg
           35                  40                  45
   Asn Pro Asn Tyr Lys Phe Asn Gly Pro Leu Ser Val Lys Lys Thr Tyr
       50                  55                  60
   Leu Lys Tyr Gly Val Pro Ile Pro Ala Trp Leu Glu Asp Ala Val Gln
   65                  70                  75                  80
   Asn Ser Thr Ser Gly Leu Ala Glu Arg
                   85
(2) INFORMATION FOR SEQ ID NO:3:
   (i) SEQUENCE CHARACTERISTICS:
      (A) LENGTH: 24 amino acids
      (B) TYPE: amino acid
      (D) TOPOLOGY: linear
   (ii) MOLECULE TYPE: peptide
   (vii) IMMEDIATE SOURCE:
      (B) CLONE: signal peptide
   (xi) SEQUENCE DESCRIPTION: SEQ ID NO:3:
   Met Arg Arg Thr Ser Lys Leu Thr Thr Phe Ser Leu Leu Phe Ser Leu
   1               5                   10                  15
   Val Leu Leu Ser Ala Ala Leu Ala
               20
(2) INFORMATION FOR SEQ ID NO:4:
   (i) SEQUENCE CHARACTERISTICS:
      (A) LENGTH: 22 amino acids
      (B) TYPE: amino acid
      (D) TOPOLOGY: linear
   (ii) MOLECULE TYPE: peptide
   (vii) IMMEDIATE SOURCE:
      (B) CLONE: signal peptide
   (xi) SEQUENCE DESCRIPTION: SEQ ID NO:4:
   Met Leu Ser Phe Val Lys Lys Ser Ile Ala Leu Val Ala Ala Leu Gln
   1               5                   10                  15
   Ala Val Thr Ala Leu Ala
               20
(2) INFORMATION FOR SEQ ID NO:5:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (xi) SEQUENCE DESCRIPTION: SEQ ID NO:5:
   Thr Pro Ile Ser Ser Glu Ala Gly Val Glu Lys Arg
   1               5                   10
(2) INFORMATION FOR SEQ ID NO:6:
   (i) SEQUENCE CHARACTERISTICS:
      (A) LENGTH: 1167 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sequence coding for SEQ ID NO: 1
   (xi) SEQUENCE DESCRIPTION: SEQ ID NO:6:
   GGTAGTGGTT TTGCAAATGC CGTCTACTTC ACCAACTGGG GCATTTATGG CCGCAACTTC    60
   CAGCCTGCCG ACCTTCCTGC CTCGGAGATT ACTCACGTAC TCTACTCCTT CATGAATGTC   120
   CGCGCAGATG GCACCATCTT TTCCGGTGAT ACCTATGCCG ACTACGAGAA GCACTACGCT   180
   GGTGACTCTT GGAACGATGT GGGCACGAAC GCTTACGGTT GTGTTAAGCA ACTTTATCTT   240
   CTCAAGAAGC AGAACCGCAA CATGAAGGTG ATGCTGTCGA TTGGTGGTTG GACATGGTCT   300
   ACCAACTTCC CCGCTGCCGC CAGCTCGGCT GCTACCCGAA AGACTTTTGC TCAGTCTGCT   360
   GTTGGCTTCA TGAAGGACTG GGGTTTCGAC GGTATTGATA TCGACTGGGA GTACCCCGCC   420
   GATGCCACTC AGGCTCAGAA TATGGTTCTC TTGCTACAGG CTGTCCGCAG TGAGCTCGAC   480
   TCCTACGCTG CCCAGTACGC CAAGGGTCAC CACTTCCTGC TTTCAATTGC CGCCCCTGCT   540
   GGACCTGACA ATTATAACAA GCTGAAGTTT GCTGAGCTTG GCAAGGTTCT CGATTACATT   600
   AACCTCATGG CTTACGATTA CGCTGGATCT TGGAGCAACT ACACTGGCCA CGATGCCAAC   660
   ATATACGCAA ACCCGCAGAA CCCCAACGCC ACCCCTTACA ACACGGACGA TGCTGTCCAG   720
   GCCTATATCA ACGGCGGCGT CCCTGCCAAC AAGATCGTCC TTGGTATGCC AATCTACGGC   780
   CGATCCTTCC AGCAAACCGA GGGTATCGGT AAGCCTTACA ATGGTATTGG CTCTGGTAGC   840
   TGGGAGAACG GTATCTGGGA CTACAAGGCT CTCCCCAAGG CTGGTGCCAC CGTCAAGTGC   900
   GACGATACCG CCAAGGGATG CTACAGCTAC GATCCAAGCA CTAAGGAGCT TATTTCTTTC   960
   GATACGCCGG CTATGATCAG CACCAAAGTT AGCTGGCTCA AGGGCAAGGG CCTTGGCGGC  1020
   AGCATGTTCT GGGAGGCTTC TGCCGACAAG AAGGGCTCGG ACTCTCTTAT TAGCACCAGC  1080
   CACCAAGGTC TCGGTAGCCA GGACAGCACT CAGAACTACC TCGACTACCC TAACTCCAAG  1140
   TACGACAACA TCAAGAAGGG CATGAAC                                       1167
(2) INFORMATION FOR SEQ ID NO:7:
   (i) SEQUENCE CHARACTERISTICS:
      (A) LENGTH: 66 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sequence coding for SEQ ID NO: 4
   (xi) SEQUENCE DESCRIPTION: SEQ ID NO:7:
   ATGTTGAGCT TTGTCAAAAA GTCGATCGCC TTGGTGGCGG CCCTGCAGGC GGTCACTGCC    60
   CTGGCC                                                               66
(2) INFORMATION FOR SEQ ID NO:8:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sequence coding for SEQ ID NO: 5
   (xi) SEQUENCE DESCRIPTION: SEQ ID NO:8:
   ACGCCAATCT CCAGTGAAGC TGGTGTTGAG AAGCGC 36
(2) INFORMATION FOR SEQ ID NO:9:
   (i) SEQUENCE CHARACTERISTICS:
      (A) LENGTH: 1405 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: sig_peptide
      (B) LOCATION: 97..198
   (ix) FEATURE:
      (A) NAME/KEY: mat_peptide
      (B) LOCATION: 199..1365
   (ix) FEATURE:
      (A) NAME/KEY: CDS
      (B) LOCATION: 97..1365
   (xi) SEQUENCE DESCRIPTION: SEQ ID NO:9:
   AGCAACTCAA TAGGTACAAG CCTAACAGCA TAGCTCCCTC TAGAGTCAGC ACGCCGAATC      60
   AGTTGATTCT CTACAACCTT CTGTACCTCA ACTACT ATG TTG AGC TTT GTC AAA      114
                                           Met Leu Ser Phe Val Lys
                                           -34             -30
   AAG TCG ATC GCC TTG GTG GCG GCC CTG CAG GCG GTC ACT GCC CTG GCC      162
   Lys Ser Ile Ala Leu Val Ala Ala Leu Gln Ala Val Thr Ala Leu Ala
               -25                 -20                 -15
   ACG CCA ATC TCC AGT GAA GCT GGT GTT GAG AAG CGC GGT AGT GGT TTT      210
   Thr Pro Ile Ser Ser Glu Ala Gly Val Glu Lys Arg Gly Ser Gly Phe
           -10                 -5                  1
   GCA AAT GCC GTC TAC TTC ACC AAC TGG GGC ATT TAT GGC CGC AAC TTC      258
   Ala Asn Ala Val Tyr Phe Thr Asn Trp Gly Ile Tyr Gly Arg Asn Phe
   5                   10                  15                  20
   CAG CCT GCC GAC CTT CCT GCC TCG GAG ATT ACT CAC GTA CTC TAC TCC      306
   Gln Pro Ala Asp Leu Pro Ala Ser Glu Ile Thr His Val Leu Tyr Ser
                   25                  30                  35
   TTC ATG AAT GTC CGC GCA GAT GGC ACC ATC TTT TCC GGT GAT ACC TAT      354
   Phe Met Asn Val Arg Ala Asp Gly Thr Ile Phe Ser Gly Asp Thr Tyr
               40                  45                  50
   GCC GAC TAC GAG AAG CAC TAC GCT GGT GAC TCT TGG AAC GAT GTG GGC      402
   Ala Asp Tyr Glu Lys His Tyr Ala Gly Asp Ser Trp Asn Asp Val Gly
           55                  60                  65
   ACG AAC GCT TAC GGT TGT GTT AAG CAA CTT TAT CTT CTC AAG AAG CAG      450
   Thr Asn Ala Tyr Gly Cys Val Lys Gln Leu Tyr Leu Leu Lys Lys Gln
       70                  75                  80
   AAC CGC AAC ATG AAG GTG ATG CTG TCG ATT GGT GGT TGG ACA TGG TCT      498
   Asn Arg Asn Met Lys Val Met Leu Ser Ile Gly Gly Trp Thr Trp Ser
   85                  90                  95                  100
   ACC AAC TTC CCC GCT GCC GCC AGC TCG GCT GCT ACC CGA AAG ACT TTT      546
   Thr Asn Phe Pro Ala Ala Ala Ser Ser Ala Ala Thr Arg Lys Thr Phe
                   105                 110                 115
   GCT CAG TCT GCT GTT GGC TTC ATG AAG GAC TGG GGT TTC GAC GGT ATT      594
   Ala Gln Ser Ala Val Gly Phe Met Lys Asp Trp Gly Phe Asp Gly Ile
               120                 125                 130
   GAT ATC GAC TGG GAG TAC CCC GCC GAT GCC ACT CAG GCT CAG AAT ATG      642
   Asp Ile Asp Trp Glu Tyr Pro Ala Asp Ala Thr Gln Ala Gln Asn Met
           135                 140                 145
   GTT CTC TTG CTA CAG GCT GTC CGC AGT GAG CTC GAC TCC TAC GCT GCC      690
   Val Leu Leu Leu Gln Ala Val Arg Ser Glu Leu Asp Ser Tyr Ala Ala
       150                 155                 160
   CAG TAC GCC AAG GGT CAC CAC TTC CTG CTT TCA ATT GCC GCC CCT GCT      738
   Gln Tyr Ala Lys Gly His His Phe Leu Leu Ser Ile Ala Ala Pro Ala
   165                 170                 175                 180
   GGA CCT GAC AAT TAT AAC AAG CTG AAG TTT GCT GAG CTT GGC AAG GTT      786
   Gly Pro Asp Asn Tyr Asn Lys Leu Lys Phe Ala Glu Leu Gly Lys Val
                   185                 190                 195
   CTC GAT TAC ATT AAC CTC ATG GCT TAC GAT TAC GCT GGA TCT TGG AGC      834
   Leu Asp Tyr Ile Asn Leu Met Ala Tyr Asp Tyr Ala Gly Ser Trp Ser
               200                 205                 210
   AAC TAC ACT GGC CAC GAT GCC AAC ATA TAC GCA AAC CCG CAG AAC CCC      882
   Asn Tyr Thr Gly His Asp Ala Asn Ile Tyr Ala Asn Pro Gln Asn Pro
           215                 220                 225
   AAC GCC ACC CCT TAC AAC ACG GAC GAT GCT GTC CAG GCC TAT ATC AAC         930
   Asn Ala Thr Pro Tyr Asn Thr Asp Asp Ala Val Gln Ala Tyr Ile Asn
       230                 235                 240
   GGC GGC GTC CCT GCC AAC AAG ATC GTC CTT GGT ATG CCA ATC TAC GGC         978
   Gly Gly Val Pro Ala Asn Lys Ile Val Leu Gly Met Pro Ile Tyr Gly
   245                 250                 255                 260
   CGA TCC TTC CAG CAA ACC GAG GGT ATC GGT AAG CCT TAC AAT GGT ATT        1026
   Arg Ser Phe Gln Gln Thr Glu Gly Ile Gly Lys Pro Tyr Asn Gly Ile
                   265                 270                 275
   GGC TCT GGT AGC TGG GAG AAC GGT ATC TGG GAC TAC AAG GCT CTC CCC        1074
   Gly Ser Gly Ser Trp Glu Asn Gly Ile Trp Asp Tyr Lys Ala Leu Pro
               280                 285                 290
   AAG GCT GGT GCC ACC GTC AAG TGC GAC GAT ACC GCC AAG GGA TGC TAC        1122
   Lys Ala Gly Ala Thr Val Lys Cys Asp Asp Thr Ala Lys Gly Cys Tyr
           295                 300                 305
   AGC TAC GAT CCA AGC ACT AAG GAG CTT ATT TCT TTC GAT ACG CCG GCT        1170
   Ser Tyr Asp Pro Ser Thr Lys Glu Leu Ile Ser Phe Asp Thr Pro Ala
       310                 315                 320
   ATG ATC AGC ACC AAA GTT AGC TGG CTC AAG GGC AAG GGC CTT GGC GGC        1218
   Met Ile Ser Thr Lys Val Ser Trp Leu Lys Gly Lys Gly Leu Gly Gly
   325                 330                 335                 340
   AGC ATG TTC TGG GAG GCT TCT GCC GAC AAG AAG GGC TCG GAC TCT CTT        1266
   Ser Met Phe Trp Glu Ala Ser Ala Asp Lys Lys Gly Ser Asp Ser Leu
                   345                 350                 355
   ATT AGC ACC AGC CAC CAA GGT CTC GGT AGC CAG GAC AGC ACT CAG AAC        1314
   Ile Ser Thr Ser His Gln Gly Leu Gly Ser Gln Asp Ser Thr Gln Asn
               360                 365                 370
   TAC CTC GAC TAC CCT AAC TCC AAG TAC GAC AAC ATC AAG AAG GGC ATG        1362
   Tyr Leu Asp Tyr Pro Asn Ser Lys Tyr Asp Asn Ile Lys Lys Gly Met
           375                 380                 385
   AAC TAAGCAGTCG GTGTTTGCAT AGCTTGATTG ATGCTCGAGG                        1405
   Asn
(2) INFORMATION FOR SEQ ID NO:10:
   (i) SEQUENCE CHARACTERISTICS:
      (A) LENGTH: 423 amino acids
      (B) TYPE: amino acid
      (D) TOPOLOGY: linear
   (ii) MOLECULE TYPE: protein
   (xi) SEQUENCE DESCRIPTION: SEQ ID NO:10:
   Met Leu Ser Phe Val Lys Lys Ser Ile Ala Leu Val Ala Ala Leu Gln
   -34             -30                 -25                 -20
   Ala Val Thr Ala Leu Ala Thr Pro Ile Ser Ser Glu Ala Gly Val Glu
               -15                 -10                 -5
   Lys Arg Gly Ser Gly Phe Ala Asn Ala Val Tyr Phe Thr Asn Trp Gly
           1               5                   10
   Ile Tyr Gly Arg Asn Phe Gln Pro Ala Asp Leu Pro Ala Ser Glu Ile
   15                  20                  25                  30
   Thr His Val Leu Tyr Ser Phe Met Asn Val Arg Ala Asp Gly Thr Ile
                   35                  40                  45
   Phe Ser Gly Asp Thr Tyr Ala Asp Tyr Glu Lys His Tyr Ala Gly Asp
               50                  55                  60
   Ser Trp Asn Asp Val Gly Thr Asn Ala Tyr Gly Cys Val Lys Gln Leu
           65                  70                  75
   Tyr Leu Leu Lys Lys Gln Asn Arg Asn Met Lys Val Met Leu Ser Ile
       80                  85                  90
   Gly Gly Trp Thr Trp Ser Thr Asn Phe Pro Ala Ala Ala Ser Ser Ala
   95                  100                 105                 110
   Ala Thr Arg Lys Thr Phe Ala Gln Ser Ala Val Gly Phe Met Lys Asp
                   115                 120                 125
   Trp Gly Phe Asp Gly Ile Asp Ile Asp Trp Glu Tyr Pro Ala Asp Ala
               130                 135                 140
   Thr Gln Ala Gln Asn Met Val Leu Leu Leu Gln Ala Val Arg Ser Glu
           145                 150                 155
   Leu Asp Ser Tyr Ala Ala Gln Tyr Ala Lys Gly His His Phe Leu Leu
       160                 165                 170
   Ser Ile Ala Ala Pro Ala Gly Pro Asp Asn Tyr Asn Lys Leu Lys Phe
   175                 180                 185                 190
   Ala Glu Leu Gly Lys Val Leu Asp Tyr Ile Asn Leu Met Ala Tyr Asp
                   195                 200                 205
   Tyr Ala Gly Ser Trp Ser Asn Tyr Thr Gly His Asp Ala Asn Ile Tyr
               210                 215                 220
   Ala Asn Pro Gln Asn Pro Asn Ala Thr Pro Tyr Asn Thr Asp Asp Ala
           225                 230                 235
   Val Gln Ala Tyr Ile Asn Gly Gly Val Pro Ala Asn Lys Ile Val Leu
       240                 245                 250
   Gly Met Pro Ile Tyr Gly Arg Ser Phe Gln Gln Thr Glu Gly Ile Gly
   255                 260                 265                 270
   Lys Pro Tyr Asn Gly Ile Gly Ser Gly Ser Trp Glu Asn Gly Ile Trp
                   275                 280                 285
   Asp Tyr Lys Ala Leu Pro Lys Ala Gly Ala Thr Val Lys Cys Asp Asp
               290                 295                 300
   Thr Ala Lys Gly Cys Tyr Ser Tyr Asp Pro Ser Thr Lys Glu Leu Ile
           305                 310                 315
   Ser Phe Asp Thr Pro Ala Met Ile Ser Thr Lys Val Ser Trp Leu Lys
       320                 325                 330
   Gly Lys Gly Leu Gly Gly Ser Met Phe Trp Glu Ala Ser Ala Asp Lys
   335                 340                 345                 350
   Lys Gly Ser Asp Ser Leu Ile Ser Thr Ser His Gln Gly Leu Gly Ser
                   355                 360                 365
   Gln Asp Ser Thr Gln Asn Tyr Leu Asp Tyr Pro Asn Ser Lys Tyr Asp
               370                 375                 380
   Asn Ile Lys Lys Gly Met Asn
           385
(2) INFORMATION FOR SEQ ID NO:11:
   (i) SEQUENCE CHARACTERISTICS:
      (A) LENGTH: 1701 base pairs
      (B) TYPE: nucleic acid
      (C) STRANDEDNESS: single
      (D) TOPOLOGY: linear
   (ii) MOLECULE TYPE: DNA (genomic)
   (ix) FEATURE:
      (A) NAME/KEY: intron
      (B) LOCATION: 266..320
      (D) OTHER INFORMATION: /number= 1
   (ix) FEATURE:
      (A) NAME/KEY: intron
      (B) LOCATION: 420..472
      (D) OTHER INFORMATION: /number= 2
   (ix) FEATURE:
      (A) NAME/KEY: intron
      (B) LOCATION: 523..571
      (D) OTHER INFORMATION: /number= 3
   (ix) FEATURE:
      (A) NAME/KEY: CDS
      (B) LOCATION: join(126..265, 321. .419, 473..522, 572..1551)
   (ix) FEATURE:
      (A) NAME/KEY: sig_peptide
      (B) LOCATION: 126..227
   (ix) FEATURE:
      (A) NAME/KEY: mat_peptide
      (B) LOCATION: join(228..265, 321..419, 473..522, 572..1551)
   (xi) SEQUENCE DESCRIPTION: SEQ ID NO:11:
(2) INFORMATION FOR SEQ ID NO:12:
   (i) SEQUENCE CHARACTERISTICS:
      (A) LENGTH: 423 amino acids
      (B) TYPE: amino acid
      (D) TOPOLOGY: linear
   (ii) MOLECULE TYPE: protein
   (xi) SEQUENCE DESCRIPTION: SEQ ID NO:12:
   Met Leu Ser Phe Val Lys Lys Ser Ile Ala Leu Val Ala Ala Leu Gln
   -34             -30                 -25                 -20
   Ala Val Thr Ala Leu Ala Thr Pro Ile Ser Ser Glu Ala Gly Val Glu
               -15                 -10                  -5
   Lys Arg Gly Ser Gly Phe Ala Asn Ala Val Tyr Phe Thr Asn Trp Gly
             1               5                  10
   Ile Tyr Gly Arg Asn Phe Gln Pro Ala Asp Leu Pro Ala Ser Glu Ile
    15                  20                  25                  30
   Thr His Val Leu Tyr Ser Phe Met Asn Val Arg Ala Asp Gly Thr Ile
                   35                  40                  45
   Phe Ser Gly Asp Thr Tyr Ala Asp Tyr Glu Lys His Tyr Ala Gly Asp
               50                  55                  60
   Ser Trp Asn Asp Val Gly Thr Asn Ala Tyr Gly Cys Val Lys Gln Leu
           65                  70                  75
   Tyr Leu Leu Lys Lys Gln Asn Arg Asn Met Lys Val Met Leu Ser Ile
       80                  85                  90
   Gly Gly Trp Thr Trp Ser Thr Asn Phe Pro Ala Ala Ala Ser Ser Ala
   95                  100                 105                 110
   Ala Thr Arg Lys Thr Phe Ala Gln Ser Ala Val Gly Phe Met Lys Asp
                   115                 120                 125
   Trp Gly Phe Asp Gly Ile Asp Ile Asp Trp Glu Tyr Pro Ala Asp Ala
               130                 135                 140
   Thr Gln Ala Gln Asn Met Val Leu Leu Leu Gln Ala Val Arg Ser Glu
           145                 150                 155
   Leu Asp Ser Tyr Ala Ala Gln Tyr Ala Lys Gly His His Phe Leu Leu
       160                 165                 170
   Ser Ile Ala Ala Pro Ala Gly Pro Asp Asn Tyr Asn Lys Leu Lys Phe
   175                 180                 185                 190
   Ala Glu Leu Gly Lys Val Leu Asp Tyr Ile Asn Leu Met Ala Tyr Asp
                   195                 200                 205
   Tyr Ala Gly Ser Trp Ser Asn Tyr Thr Gly His Asp Ala Asn Ile Tyr
               210                 215                 220
   Ala Asn Pro Gln Asn Pro Asn Ala Thr Pro Tyr Asn Thr Asp Asp Ala
           225                 230                 235
   Val Gln Ala Tyr Ile Asn Gly Gly Val Pro Ala Asn Lys Ile Val Leu
       240                 245                 250
   Gly Met Pro Ile Tyr Gly Arg Ser Phe Gln Gln Thr Glu Gly Ile Gly
   255                 260                 265                 270
   Lys Pro Tyr Asn Gly Ile Gly Ser Gly Ser Trp Glu Asn Gly Ile Trp
                   275                 280                 285
   Asp Tyr Lys Ala Leu Pro Lys Ala Gly Ala Thr Val Lys Cys Asp Asp
               290                 295                 300
   Thr Ala Lys Gly Cys Tyr Ser Tyr Asp Pro Ser Thr Lys Glu Leu Ile
           305                 310                 315
   Ser Phe Asp Thr Pro Ala Met Ile Ser Thr Lys Val Ser Trp Leu Lys
       320                 325                 330
   Gly Lys Gly Leu Gly Gly Ser Met Phe Trp Glu Ala Ser Ala Asp Lys
   335                 340                 345                 350
   Lys Gly Ser Asp Ser Leu Ile Ser Thr Ser His Gln Gly Leu Gly Ser
                   355                 360                 365
   Gln Asp Ser Thr Gln Asn Tyr Leu Asp Tyr Pro Asn Ser Lys Tyr Asp
               370                 375                 380
   Asn Ile Lys Lys Gly Met Asn
           385
(2) INFORMATION FOR SEQ ID NO:13:
   (i) SEQUENCE CHARACTERISTICS:
      (A) LENGTH: 1364 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
   GGATCCGCTA ACTGACATCG ATATACACAA TGTTGAGCTT TGTCAAAAAG TCGATCGCCT     60
   TGGTGGCGGC CCTGCAGGCG GTCACTGCCC TGGCCACGCC AATCTCCAGT GAAGCTGGTG    120
   TTGAGAAGCG CGGTAGTGGT TTTGCAAATG CCGTCTACTT CACCAACTGG GGCATTTATG    180
   GCCGCAACTT CCAGCCTGCC GACCTTCCTG CCTCGGAGAT TACTCACGTA CTCTACTCCT    240
   TCATGAATGT CCGCGCAGAT GGCACCATCT TTTCCGGTGA TACCTATGCC GACTACGAGA    300
   AGCACTACGC TGGTGACTCT TGGAACGATG TGGGCACGAA CGCTTACGGT TGTGTTAAGC    360
   AACTTTATCT TCTCAAGAAG CAGAACCGCA ACATGAAGGT GATGCTGTCG ATTGGTGGTT    420
   GGACATGGTC TACCAACTTC CCCGCTGCCG CCAGCTCGGC TGCTACCCGA AAGACTTTTG    480
   CTCAGTCTGC TGTTGGCTTC ATGAAGGACT GGGGTTTCGA CGGTATTGAT ATCGACTGGG    540
   AGTACCCCGC CGATGCCACT CAGGCTCAGA ATATGGTTCT CTTGCTACAG GCTGTCCGCA    600
   GTGAGCTCGA CTCCTACGCT GCCCAGTACG CCAAGGGTCA CCACTTCCTG CTTTCAATTG    660
   CCGCCCCTGC TGGACCTGAC AATTATAACA AGCTGAAGTT TGCTGAGCTT GGCAAGGTTC    720
   TCGATTACAT TAACCTCATG GCTTACGATT ACGCTGGATC TTGGAGCAAC TACACTGGCC    780
   ACGATGCCAA CATATACGCA AACCCGCAGA ACCCCAACGC CACCCCTTAC AACACGGACG    840
   ATGCTGTCCA GGCCTATATC AACGGCGGCG TCCCTGCCAA CAAGATCGTC CTTGGTATGC    900
   CAATCTACGG CCGATCCTTC CAGCAAACCG AGGGTATCGG TAAGCCTTAC AATGGTATTG    960
   GCTCTGGTAG CTGGGAGAAC GGTATCTGGG ACTACAAGGC TCTCCCCAAG GCTGGTGCCA   1020
   CCGTCAAGTG CGACGATACC GCCAAGGGAT GCTACAGCTA CGATCCAAGC ACTAAGGAGC   1080
   TTATTTCTTT CGATACGCCG GCTATGATCA GCACCAAAGT TAGCTGGCTC AAGGGCAAGG   1140
   GCCTTGGCGG CAGCATGTTC TGGGAGGCTT CTGCCGACAA GAAGGGCTCG GACTCTCTTA   1200
   TTAGCACCAG CCACCAAGGT CTCGGTAGCC AGGACAGCAC TCAGAACTAC CTCGACTACC   1260
   CTAACTCCAA GTACGACAAC ATCAAGAAGG GCATGAACTA AGCAGTCGGT GTTTGCATAG   1320
   CTTGATTGAT GCTCGACTCT AGAGGATCGA ACTGTACCGA GCTC                     1364
(2) INFORMATION FOR SEQ ID NO:14:
   (i) SEQUENCE CHARACTERISTICS:
      (A) LENGTH: 1320 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   GGATCCATGA AGAAGAATAG GATGATGATG ATGATATGGA GCGTAGGAGT GGTGTGGATG     60
   CTGTTGTTGG TTGGAGGAAG CTACGGAGGT AGTGGTTTTG CAAATGCCGT CTACTTCACC    120
   AACTGGGGCA TTTATGGCCG CAACTTCCAG CCTGCCGACC TTCCTGCCTC GGAGATTACT    180
   CACGTACTCT ACTCCTTCAT GAATGTCCGC GCAGATGGCA CCATCTTTTC CGGTGATACC    240
   TATGCCGACT ACGAGAAGCA CTACGCTGGT GACTCTTGGA ACGATGTGGG CACGAACGCT    300
   TACGGTTGTG TTAAGCAACT TTATCTTCTC AAGAAGCAGA ACCGCAACAT GAAGGTGATG    360
   CTGTCGATTG GTGGTTGGAC ATGGTCTACC AACTTCCCCG CTGCCGCCAG CTCGGCTGCT    420
   ACCCGAAAGA CTTTTGCTCA GTCTGCTGTT GGCTTCATGA AGGACTGGGG TTTCGACGGT    480
   ATTGATATCG ACTGGGAGTA CCCCGCCGAT GCCACTCAGG CTCAGAATAT GGTTCTCTTG    540
   CTACAGGCTG TCCGCAGTGA GCTCGACTCC TACGCTGCCC AGTACGCCAA GGGTCACCAC    600
   TTCCTGCTTT CAATTGCCGC CCCTGCTGGA CCTGACAATT ATAACAAGCT GAAGTTTGCT    660
   GAGCTTGGCA AGGTTCTCGA TTACATTAAC CTCATGGCTT ACGATTACGC TGGATCTTGG    720
   AGCAACTACA CTGGCCACGA TGCCAACATA TACGCAAACC CGCAGAACCC CAACGCCACC    780
   CCTTACAACA CGGACGATGC TGTCCAGGCC TATATCAACG GCGGCGTCCC TGCCAACAAG    840
   ATCGTCCTTG GTATGCCAAT CTACGGCCGA TCCTTCCAGC AAACCGAGGG TATCGGTAAG    900
   CCTTACAATG GTATTGGCTC TGGTAGCTGG GAGAACGGTA TCTGGGACTA CAAGGCTCTC    960
   CCCAAGGCTG GTGCCACCGT CAAGTGCGAC GATACCGCCA AGGGATGCTA CAGCTACGAT   1020
   CCAAGCACTA AGGAGCTTAT TTCTTTCGAT ACGCCGGCTA TGATCAGCAC CAAAGTTAGC   1080
   TGGCTCAAGG GCAAGGGCCT TGGCGGCAGC ATGTTCTGGG AGGCTTCTGC CGACAAGAAG   1140
   GGCTCGGACT CTCTTATTAG CACCAGCCAC CAAGGTCTCG GTAGCCAGGA CAGCACTCAG   1200
   AACTACCTCG ACTACCCTAA CTCCAAGTAC GACAACATCA AGAAGGGCAT GAACTAAGCA   1260
   GTCGGTGTTT GCATAGCTTG ATTGATGCTC GACTCTAGAG GATCGAACTG TACCGAGCTC   1320
(2) INFORMATION FOR SEQ ID NO:15:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: misc_feature
      (B) LOCATION: 3
      (D) OTHER INFORMATION: /note= "N = inosine"
   (ix) FEATURE:
      (A) NAME/KEY: misc_feature
      (B) LOCATION: 9
      (D) OTHER INFORMATION: /note= "N = inosine"
   (ix) FEATURE:
      (A) NAME/KEY: misc_feature
      (B) LOCATION: 15
      (D) OTHER INFORMATION: /note= "N = inosine"
   (ix) FEATURE:
      (A) NAME/KEY: misc_feature
      (B) LOCATION: 18
      (D) OTHER INFORMATION: /note= "N = inosine"
   (xi) SEQUENCE DESCRIPTION: SEQ ID NO:15:
   GGNTTYGCNA AYGCNGTNTA YTTYAC 26
(2) INFORMATION FOR SEQ ID NO:16:
   (i) SEQUENCE CHARACTERISTICS:
      (A) LENGTH: 8 amino acids
      (B) TYPE: amino acid
      (D) TOPOLOGY: linear
   (ii) MOLECULE TYPE: protein
   (xi) SEQUENCE DESCRIPTION: SEQ ID NO:16:
   Gly Phe Ala Asn Ala Val Tyr Phe
   1 5
(2) INF0RMATION FOR SEQ ID NO:17:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
   CAAATGCCGT CTACTTCACC 20
(2) INFORMATION FOR SEQ ID NO:19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
   CCTCATGGCT TACGATTACG 20
(2) INFORMATION FOR SEQ ID NO:20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
   TCCAACCTCG AGCATCAATC 20
(2) INFORMATION FOR SEQ ID NO:21:
   (i) SEQUENCE CHARACTERISTICS:
      (A) LENGTH: 34 amino acids
      (B) TYPE: amino acid
      (D) TOPOLOGY: linear
   (ii) MOLECULE TYPE: peptide
   (xi) SEQUENCE DESCRIPTION: SEQ ID NO:21:
   Met Leu Ser Phe Val Lys Lys Ser Ile Ala Leu Val Ala Ala Leu Gln
   1               5                   10                  15
   Ala Val Thr Ala Leu Ala Thr Pro Ile Ser Ser Glu Ala Gly Val Glu
               20                  25                  30
   Lys Arg
(2) INFORMATION FOR SEQ ID NO:22:
   (i) SEQUENCE CHARACTERISTICS:
      (A) LENGTH: 27 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:22:
   TATGGGTAGT GGTTTTGCAA ATGCCGT                                    27
(2) INFORMATION FOR SEQ ID NO:23:
   (i) SEQUENCE CHARACTERISTICS:
      (A) LENGTH: 38 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:23:
   AGCTTGGATA AAAGAGGTAG TGGTTTTGCA AATGCCGT                        38
(2) INFORMATION FOR SEQ ID NO:24:
   (i) SEQUENCE CHARACTERISTICS:
      (A) LENGTH: 58 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:24:
   CGATATACAC AATGTTGAGC TTTGTCAAAA AGTCGATCGC CTTGGTGGCG GCCCTGCA 58
(2) INFORMATION FOR SEQ ID NO:25:
   (i) SEQUENCE CHARACTERISTICS:
      (A) LENGTH: 17 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:25:
   GATCCGCTAA CTGACAT 17
(2) INFORMATION FOR SEQ ID NO:26:
   (i) SEQUENCE CHARACTERISTICS:
      (A) LENGTH: 19 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:26:
   GATCGAACTG TACCGAGCT 19
(2) INFORMATION FOR SEQ ID NO:27:
   (i) SEQUENCE CHARACTERISTICS:
      (A) LENGTH: 109 base pairs
      (B) TYPE: nucleic acid
      (C) STRANDEDNESS: double
      (D) TOPOLOGY: linear
   (ii) MOLECULE TYPE: cDNA
   (xi) SEQUENCE DESCRIPTION: SEQ ID NO:27:
   GATCCATGAA GAAGAATAGG ATGATGATGA TGATATGGAG CGTAGGAGTG GTGTGGATGC    60
   TGTTGTTGGT TGGAGGAAGC TACGGAGGTA GTGGTTTTGC AAATGCCGT               109
(2) INFORMATION FOR SEQ ID NO:28:
   (i) SEQUENCE CHARACTERISTICS:
      (A) LENGTH: 27 amino acids
      (B) TYPE: amino acid
      (D) T0POLOGY: linear
   (ii) MOLECULE TYPE: peptide
   (xi) SEQUENCE DESCRIPTION: SEQ ID NO:28:
   Met Lys Lys Asn Arg Met Met Met Met Ile Trp Ser Val Gly Val Val
   1               5                   10                  15
   Trp Met Leu Leu Leu Val Gly Gly Ser Tyr Gly
               20                  25
(2) INFORMATION FOR SEQ ID NO:29:
   (i) SEQUENCE CHARACTERISTICS:
      (A) LENGTH: 81 base pairs
      (B) TYPE: nucleic acid
      (C) STRANDEDNESS: single
      (D) TOPOLOGY: linear
   (ii) MOLECULE TYPE: cDNA
   (vii) IMMEDIATE SOURCE:
      (B) CLONE: sequence coding for SEQ ID NO: 28
   (xi) SEQUENCE DESCRIPTION: SEQ ID NO:29:
   ATGAAGAAGA ATAGGATGAT GATGATGATA TGGAGCGTAG GAGTGGTGTG GATGCTGTTG    60
   TTGGTTGGAG GAAGCTACGG A                                              81